(19) Europäisches Patentamt
European Patent Office
Office européen des brevets

(11) **EP 3 295 988 B1**

(12) **EUROPEAN PATENT SPECIFICATION**

(45) Date of publication and mention
of the grant of the patent:
**24.02.2021 Bulletin 2021/08**

(51) Int Cl.:
*A61M 25/10* *(2013.01)*          *A61B 18/12* *(2006.01)*
*A61B 5/00* *(2006.01)*

(21) Application number: **16792141.0**

(22) Date of filing: **06.05.2016**

(86) International application number:
**PCT/CN2016/081353**

(87) International publication number:
**WO 2016/180291 (17.11.2016 Gazette 2016/46)**

(54) **ENDOSCOPIC OCT MICROPROBE, OCT IMAGING SYSTEM, AND USE METHOD**

ENDOSKOPISCHE OCT-MIKROSONDE, OCT-BILDGEBUNGSSYSTEM UND VERWENDUNGSVERFAHREN

MICROSONDE ENDOSCOPIQUE OCT, SYSTÈME D'IMAGERIE OCT, ET SON PROCÉDÉ D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.05.2015 CN 201510234798**

(43) Date of publication of application:
**21.03.2018 Bulletin 2018/12**

(73) Proprietor: **Micro-Tech (Nanjing) Co., Ltd.**
**Nanjing, Jiangsu 210000 (CN)**

(72) Inventors:
• **XI, Jiefeng**
**Nanjing**
**Jiangsu 210000 (CN)**
• **GAO, Duangui**
**Nanjing**
**Jiangsu 210000 (CN)**
• **LI, Changqing**
**Nanjing**
**Jiangsu 210000 (CN)**
• **LENG, Derong**
**Nanjing**
**Jiangsu 210000 (CN)**

(74) Representative: **Viering, Jentschura & Partner mbB**
**Patent- und Rechtsanwälte**
**Hamborner Straße 53**
**40472 Düsseldorf (DE)**

(56) References cited:
WO-A1-2008/137710          WO-A2-2009/140617
CN-A- 101 032 390          CN-A- 101 600 388
CN-A- 104 248 419          CN-A- 104 794 740
CN-A- 104 799 802          CN-A- 104 825 118
CN-A- 104 825 120          CN-A- 104 825 121
CN-U- 204 636 277          CN-U- 204 636 278
CN-U- 204 671 101          CN-U- 204 698 494
CN-Y- 2 505 853            JP-A- 2007 206 049
US-A1- 2004 062 890        US-A1- 2007 219 451
US-A1- 2007 265 521        US-A1- 2008 165 366
US-A1- 2010 030 144        US-A1- 2010 292 536
US-A1- 2012 004 506        US-A1- 2013 023 760
US-A1- 2013 044 330        US-A1- 2013 204 125
US-A1- 2013 204 126        US-A1- 2014 340 756

• PREMACHANDRAN C S ET AL: "Influence of optical probe packaging on a 3D MEMS scanning micro-mirror for optical coherence tomography (OCT) applications", 58TH ELECTRONIC COMPONENTS AND TECHNOLOGY CONFERENCE - 27-30 MAY 2008 - LAKE BUENA VISTA, FL, USA, IEEE, PISCATAWAY, NJ, USA, 27 May 2008 (2008-05-27), pages 829-833, XP031276292, ISBN: 978-1-4244-2230-2
• JIEFENG XI ET AL: "Generic real-time uniform K-space sampling method for high-speed swept-Source optical coherence tomography", OPTICS EXPRESS, vol. 18, no. 9, 26 April 2010 (2010-04-26) , page 9511, XP055056201, ISSN: 1094-4087, DOI: 10.1364/OE.18.009511

## Description

### FIELD OF THE INVENTION

The present invention relates to the field of medical appliance technologies, and in particular, to an application in a human body lumen OCT scanning and imaging detection, provides an OCT microprobe and an OCT imaging system for OCT endoscopic scanning and imaging, and a method for using same.

### DESCRIPTION OF THE RELATED ART

Optical coherence tomography (OCT) has been widely used in the field of ophthalmic diagnosis. This technology is based on the optics, electronics and computer science and technology. It's a new imaging technology combining photoelectricity, high-speed data acquisition, image processing and other advanced disciplines. OCT has attracted much attention because of its high resolution, high speed imaging and so on, it's already been applied in biomedical and clinical diagnosis.

Compared to the CT, ultrasound, MRI and the others, OCT has a very high resolution. Also compared to the traditional laser confocal microscopy, the imaging depth of OCT has obvious advantages. Most of the core technologies of traditional optical probes is using fiber bundle for light transmission and imaging, or using CCD technology for imaging, nevertheless such endoscope probes can only detect the lesion on the surface of tissue, but the symptoms of early cancer usually occurred under the skin below the depth of 1-3 mm, so that the traditional optical endoscope probe seems unavailable. At present, there are endoscope probes for medical imaging by means of the ultrasound, the deep tissue information about the biological tissue under the surface can be obtained, but the resolution is only in the order of millimeters, which is easy to cause a misdiagnosis of early cancer.

With the OCT technology developing over the past decade, the endoscopic OCT technology is born and developed as a branch of OCT technology. The core goal of the endoscopic OCT technology is to miniaturize the device without reducing the resolution, and to provide high resolution OCT image for the internal organs of human body. The technology greatly expands the application field of the OCT technology, so that an object detected by OCT develops from organs on the body surface or biopsy samples to human internal organs, such as blood vessels, a alimentary tract, and a respiratory tract, and currently the technology relates to various alimentary tract lumens, such as large alimentary tract lumens (such as an esophagus and rectum) and small alimentary tract lumens (such as a biliary tract). Clinically, the OCT endoscope technology has been preliminarily applied to aspects such as detection of atherosclerosis and detection of a placement condition of vascular stents.

One of the key components in an endoscopic OCT system is an OCT microprobe. The OCT microprobe can be combined with an existing endoscope or minimally invasive technology that is clinically used, extends into internal organs of a human body, and acquires and collects backscattered light from organisms, The OCT microprobe also need to satisfy features such as a small physical size and high mechanical strength.

A grin lens as a key component of the OCT microprobe has features such as a mature processing process, low production costs, and a simple and easily assembled structure, and can also be made into a very small size and become a first choice for producing the OCT microprobe. However, the grin lens also has some defects. For example, since the numerical aperture of the grin lens is limited in its small size, the transverse resolution of the OCT microprobe is decreased, and particularly, the transverse resolution dramatically decreases when the grin lens with a long working distance.

In addition, when the OCT microprobe is used, a cylindrical plastic transparent casing is usually used for positioning and protection. When laser passes through the cylindrical plastic transparent casing, it is equivalent to passing through a cylindrical-surface negative lens, and light scattering occurs. Consequently, a laser circular facula becomes an elliptical facula, leading to deformation of a scanned image and affecting the diagnosis result. In conclusion, it is necessary to design an OCT detection system for use in blood vessels, an alimentary tract, a respiratory tract, or narrow space of human tissues. In addition, The OCT detection system also need to have a high-resolution OCT microprobe that has a relatively high transverse resolution and is capable of preventing a scanned image from deforming. Some patents including WO2008/137710A1, WO2009/140617A2, JP200720649A, US2013/204126A1, US2010/030144A1, US2007/219451A1, US2004/062890A1, US2010/292536A1, US2013/044330A1 and US2007/265521A1 are pertinent to OCT detection system. WO2008/137710A1 discloses an OCT microprobe comprising the features defined in the preamble of claim 1.

Further, the following documents are also pertinent to OCT detection:

PREMACHANDRAN C S ET AT: "Influence of optical probe packaging on a 3D MEMS scanning micromirror for optical coherence tomography (OCT) applications", 58th Electronic Components and Technology Conference 27-30 May 2008, LAKE BUENA VISTA, FL, USA, IEEE, PISACATAWAY, NJ, USA, 27 May 2008, pages 823-833;
JIEFENG XI ET AL: "Generic real-time uniform K-space sampling method for high-speed swept-Source optical coherence tomography", OPTICS EXPRESS, vol. 18, no.9, 26 April 2010, page 9511.
In this specification the following non-SI units are used, which may be converted to the respective SI or metric unit according to the following conversion:1 inch equals 2.54 centimeter.

## SUMMARY OF THE INVENTION

The invention provides an OCT microprobe as defined in claim 1, an OCT endoscopic scanning and imaging system as defined in claim 7, and a non-surgical imaging method for an OCT endoscopic imaging system as defined in claim 9. Preferred embodiments of the invention are defined in the dependent claims.

The purpose of this invention is to provide an OCT microprobe applied to OCT endoscopic scanning and imaging, the OCT microprobe comprising: a spring tube, which is capable of providing sufficient torque to keep both the distal tip and proximal tip of the probe operating synchronously when the probe with a certain length is rotating, and the single-mode fiber is transmitting inside the spring tube; a lens assembly making the light from the optical fiber gathering outside the preset working distance, comprises a glass rod and a self-focus lens,; the working distance of the OCT microprobe can be changed by changing the bonding distance between the glass rod and the single-mode fiber, and the lateral resolution of the OCT probe can be improved at the same time; the clear aperture of the self-focus lens can be increased by the bonding between the self-focus lens and the glass rod, and then the numerical aperture and lateral resolution of the OCT probe can be increased, and the physical size of the probe will be optimized. The OCT microprobe may also include a speculum, a support stainless steel tube and a grooved stainless steel tube, the tip faces of which are bonded with an optical glue.

Wherein, one side of the single-mode fiber is a standard fiber splice, the splice can connect to the rotating end of the optical fiber end in the OCT system, the single-mode fiber is inside the a spring tube (covered with a PTFE film), the spring tube can effectively protect the single-mode fiber and reduce the resistance when the probe is rotating, so that the OCT microprobe scans more smoothly, the standard fiber splice combines a support stainless steel tube, the stainless steel tube supports the OCT microprobe for scanning to make the entire rotating and scanning process more smooth. The other side of the single-mode fiber is an incline and is glued to one end face of the glass rod which is also an incline, the inclination of the surface gluing face effectively reduces the interference of the reflected light to the signal light, and the preset working distance can be obtained by changing the bonding distance between the glass rod and the single-mode fiber which will change the working distance of the OCT microprobe. And the other end of the glass rod is glued with the self-focus lens at the angle 0°end and then sealed inside the grooved stainless steel tube, by changing the length of the self-focus lens, the working distance of the OCT probe is changed to realize obtaining the best lateral resolution with the specified working distance, and the numerical aperture of the OCT probe can be increased by increasing the length of the glass rod, thereby the lateral resolution is increased as well, that means the usage of the glass rod not only increases the working distance of the microprobe, but also increases the numerical aperture of the microprobe, and the increase of the numerical aperture leads to the increase of the lateral resolution, at the same time, this design shorten the length of self-focus lens greatly and ensure that the microprobe's camber to enable the microprobe to go directly into human esophagus together with catheter through the endoscopic pliers catheter. The reflector changes forward light rays to lateral ones, and the reflector is a cylindrical-surface reflector, which could change the astigmatism influence of the protective cannula outside the OCT probe on the aggregated beam.

Preferably, the surface of the self-focus lens in contact with air is coated with an antireflection film, which could reduce reflection of light rays between optical surfaces and enhance light transmission performance. Thereby reducing the influence of the reflected light from the optical surface on the signal light and improving the sensitivity of the OCT microprobe as well; the surface of the self-focus lens in contact with air can be machined to a 4°-8° incline, which can further reduce the interference single of the light.

Preferably, an angle of the surface on which the glass rod is glued with the single-mode fiber is 4° to 12°.

Preferably, a reflective plane of the reflector faces a notch of a stainless tube and is encapsulated in the stainless tube. To reduce the impact on imaging of light scattering of a light source passing through a cylindrical inner tube, the reflector herein may be designed as a cylindrical reflector according to the inner and outer diameters of the cylindrical inner tube and the refractive index of the inner tube material. After the reflector is added, when light rays emit into the reflector, optical paths of the light rays are reflected and changed. In addition, a specially designed mirror of the reflector converges the light rays, so as to offset light scattering impact of the inner tube and correct a shape of a facula, thereby improving the imaging quality.

The other purpose of this invention is to provide an OCT endoscopic scanning and imaging system comprising: a sweep laser module, an interference module, a probe module, a data collection module, a data processing module, an image display module, an actuator, a balloon catheter, an inflation/deflation device and the OCT microprobe, wherein,

the sweep laser module comprises a high-speed sweep laser, a fiber isolator, a fiber couple.. An optical signal output from the sweep laser is isolated from a subsequent optical path, to prevent an optical signal returned by the subsequent optical path from interfering with normal operation of the laser. The interference module can use fiber-type Mach-Zehnder interferometer (MZI) or fiber-type Michelson (Michelson) interferometer structure. The MZI is mainly composed of two fiber couplers, two fiber circulator and two fiber polarization controller, in which one of the fiber couplers usually use asymmetric fiber coupler to output most of the laser to the microprobe of the laser, a fiber circulator is arranged in both the reference arm and the sample arm to collect optical signals reflected or scattered back from the two arms; the other fiber coupler use a symmetrical 2x2 fiber couple (which means a splitting ratio of 50/50) to produce an interference optical signal and reduce the DC common mode signal, the fiber polarization controller is symmetrically placed in the reference arm and the sample arm for adjusting the polarization state of the two arms to obtain the best interference optical signal. The Michelson interferometer structure is formed by a symmetric $2\times2$ fiber coupler, a fiber circulator, and two optical polarization controllers. The sweep laser passes through the fiber circular and then get into the fiber coupler, and the optical signals reflected or scattered back from the reference arm and the sample arm generate interference signal when the sweep laser passes the same fiber coupler, The fiber polarization controller is symmetrically placed in the reference arm and the sample arm to adjust the polarization state of the two arms to obtain the best interference signal. The advantages of the MZI lie in symmetry structure, simple dispersion management and high detection sensitivity. The advantages of Michelson interferometer lie in the simple structure and without introducing a polarization mode dispersion (PMD). The two interferometers are common in that an optical path difference between two arms determines a free spectral range (FSR) in which a clock signal is generated, and finally determines a maximum imaging depth of an OCT image. The probe module can be a high-speed balanced photodetector and is mainly used to convert an interference optical signal output from the interference module into an electrical signal; the data collection module is high-speed modulus collection card which is mainly used for converting the analog electrical signal into digital electrical signals, and providing the digital signals to the data processing module for digital signal processing. The data processing module is a chip (such as CPU, GPGPU, DSP, FPGA, etc.) with digital signal processing capability, which is mainly used for processing the original signals and transferring them into image signals at the end; and the image display module is mainly used for displaying the image signal and the post-processing and measurement of the image; the actuator is composed of an fiber rotating connector, a motor and an electric displacement platform, the rotating motor inside the actuator drives the OCT microprobe to rotate scanning, at the same time the electric displacement platform drives the actuator to move in a certain direction, the software will acquire the rotating scanning data and the displacement platform mobile data to reconstruct during the period, which is 3D image; the OCT microprobe is mainly used to enter the internal organs of the human body to transfer sweep laser and collect the optical signals backscattering from the biological tissue; the balloon catheter is used for expanding the lumens from the internal organs of the body, and eliminating wrinkles and stabilizing the OCT microprobe to the center of the balloon; the automatic inflation/deflation device is mainly used for dilation of balloon catheters.

Preferably, the balloon catheter comprises: a handle, where one interface of the handle is a host interface, and the other interface is a ventilation interface; a double-cavity tube, allowing an OCT optical probe to pass through; a balloon, where a front end of the balloon is blocked, and scales are provided on the balloon; an inner tube, where the length of the inner tube is determined according to the length of the balloon, and the length is less than that of the balloon; when the balloon is welded with a soft head, the balloon is pushed downwards for a certain distance, so as to be aligned with and fixed to the inner tube and then to be welded, the definition of the scanning image can be affected if the inner tube is too thick, and the rotation of the probe and concentricity can be affected if the inner tube is too thin; the inner tube is exclusively designed for the OCT probe; the inner diameter of the inner tube is 1.4 mm, and the outer diameter of the inner tube is 1.65 mm; the concentricity between the inner tube and the balloon is an offsets of no more than 500 micrometers under rated working atmospheric pressures, preferably, the rated working atmospheric pressure is 3 to 5 atmospheric pressures, and the concentricity between the inner tube and the balloon is an offset by no more than 500 micrometers under 3 atmospheric pressures; a film sleeve, located at a junction between the balloon and the double-cavity tube and control the inner tube to float within a lumen, so as to ensure that the inner tube does not offset from the center of the balloon and solve the eccentricity problem; and a soft head, which is a solid structure, where one end of the double-cavity tube is connected to the handle, the other end is connected to the inner tube and the balloon, and the other end of the balloon and the other end of the inner tube are connected to the soft head.

A conventional balloon catheter needs to be supported and guided by a guide wire. The diameter of the guide wire is usually 0.018 in, 0.035 in, 0.014 in, and 0.038 in. The balloon catheter in the present invention can pass through a 0.055 in OCT optical microprobe. Ink printing scales whose line width is less than or equal to 0.1 mm are provided on the balloon, so that a scanning direction of the microprobe can be recognized. This does not affect scanning judgment of a normal image and can also recognize a scanning position on a display screen. The front end of the balloon is blocked to prevent the entrance of body fluid, so that the impact of the body fluid on optical scanning can be prevented, and corrosion by the body fluid on precise instrument can also be prevented. In addition, the blocking material is a soft structure and does not scratch tissues and tracts of the probed object in an operation process, thereby increasing the security of the device. The soft head is a solid structure and can prevent the entrance of the body fluid.

Preferably, the rated working atmospheric pressure is 3 to 5 atmospheric pressures.

Preferably, the rated working atmospheric pressure is 3 atmospheric pressures, and a concentricity between the inner tube and the balloon is an offset of no more than 500 micrometers under 3 atmospheric pressures. The working rated pressure of the balloon is 3 atmospheric pressures, and does not destroy a normal esophagus under a relatively low pressure. In addition, the heat setting process and the welding process of the balloon can ensure that the concentricity between the inner tube and the balloon is an offset of no more than 500 micrometers under three rated atmospheric pressures, so as to facilitate optical imaging. The soft head is a slid structure and can prevent the entrance of the body fluid. The double-cavity tube is connected to the handle by using a UV adhesive, and all the other parts are connected by using a welding process. The length of the inner tube is determined according to the length of the balloon, and the length is shorter than that of the balloon; when the balloon is welded with a soft head, the balloon is pushed downwards for a certain distance, so as to be aligned with and fixed to the inner tube to be welded, so that the balloon has an elongation allowance when the balloon is filled, so as to match stretching of the inner tube and keep concentric. The welding and fixing manner of the balloon can avoid, to some degree, a problem that an optical probe is eccentric caused by relative movement of the catheter and the balloon when a patient and an organ cavity moves and a catheter is excessively inserted into the balloon in a structure in which the inner tube is not fixed and is directly inserted into the balloon. Preferably, a folding and curling temperature for the balloon is 40°C to 45°C, and the shaping time is 4h to 5 h. Compared to a conventional balloon folding process, this process can maintain memory characteristics of the balloon while ensuring the concentricity. In addition, the material of the handle in the present invention may be polycarbonate, the materials of the double-cavity tube and the soft head can be block polyether amide, and the materials of the balloon and the inner tube can be nylon and a modified polymer thereof.

A method for using a balloon catheter applied to OCT endoscopic scanning and imaging includes steps: inserting a balloon catheter with an OCT microprobe into a pliers channel; pushing a balloon to a position that needs to be scanned; then connecting one interface of a handle to an OCT device, and connecting the other interface to an inflation device for inflation; performing image scanning after inflation is completed; extracting air after the scanning is completed; and retreating the balloon catheter after air extraction is completed.

Preferably, an inflation/deflation device applied in an OCT endoscopic scanning and imaging system comprises: a sweep laser module, an interference module, a probe module, a data collection module, a data processing module, an image display module, an actuator, an OCT microprobe, a balloon catheter, and an inflation/deflation device; the inflation/deflation device is an automatic inflation/deflation device; wherein the automatic inflation/deflation device comprises a control and display module, an air pump, an inflation solenoid valve, a deflation solenoid valve, a pressure sensor, an explosion-proof pressure sensor, and a mechanical pressure switch; the automatic inflation/deflation device is applied in the OCT endoscopic scanning and imaging system to implement automatic inflation/deflation and accurate air pressure control; the air pump is connected to the throttle valve by using the inflation solenoid valve and the deflation solenoid valve; the throttle valve is connected to the balloon; there are many pressure sensors, at least one pressure sensor is connected to the balloon, at least one pressure sensor is disposed between the throttle valve, the inflation solenoid valve, and the deflation solenoid valve; the explosion-proof pressure sensor is connected with the balloon; the mechanical pressure switch is connected to the balloon; the control and display module is capable of setting the balloon pressure and the inflation time, collecting pressure, controlling the air pump to start and stop, and controlling a working condition of the solenoid valve;

a process of using the automatic inflation/deflation device comprises:

an inflation process: setting the balloon pressure and inflation time on the control and display module, the pump keeps inflating until it reaches the preset pressure value;

a deflation process: setting the balloon pressure and deflation time on the control and display module, the pump keeps deflating until it reaches the preset pressure value; and

in the inflation/deflation process, the system is under over-voltage protection by the explosion-proof pressure sensor, the air pump is closed when the balloon exceeds the preset pressure, and the pressure can be relieved through the mechanical pressure switch when the balloon exceeds the preset pressure value.

The automatic inflation/deflation device accomplishes automatic inflating and deflating, and it has a function of setting different air pressure parameters, and can perform inflation/deflation on balloons of different specifications, the inflation process can be stopped automatically when the balloon pressure reaches the preset value, and it has over-voltage protection function. It has the effects like: first of all, the manual operation of a doctor inflating/deflating a balloon is omitted, thereby shortening the inflating/deflating time for the doctor to improve the safety and avoid the risk of explosion caused by over-inflation of the balloon; secondly, the precise air pressure control guarantees the inflatable shape of the balloon consistency, since the optical imaging is sensitive to the shape of the scanned object uplifted by the balloon, the repeatability of the multiple scans for the same object is good, the doctor is able to compare the scanned image data; what's more, in the case of emergency treatment, the doctor can perform other operations while the automatic deflation is performed.

Preferably, the OCT endoscopic scanning and imaging system includes an optical clock module consisting of the interference module, the probe module, and an optical clock conversion circuit module. The interference module can use an all-fiber Mach-Zehnder Interferometer (MZI) structure which is mainly consists of two fiber couplers. A second coupler is a symmetric $2\times2$ fiber coupler. First of all, light is divided into two paths at the first fiber coupler, these two paths of light respectively pass through a first fiber and a second fiber that have a fixed optical path difference, interference occurs at the second fiber coupler. The probe module can be a high-speed balanced photodetector and is mainly used to convert an interference optical signal output from the interference module into an electrical signal. The optical interference signal generated by the MZI is converted into an electrical signal by the balanced photodetector, and then converted into an optical clock signal that is uniform in frequency domain and frequency-variable in time domain by an optical clock conversion circuit module, that is, sequentially passes through a wideband 90 degree phase shifter, two zero-crossing comparator, an exclusive-OR gate, an OR gate, and an optical clock signal output module The wideband 90 degree phase shifter is mainly used to shift a phase of an MZI electrical signal by 90 degrees, so as to increase an available spectrum interval width of an original signal, enrich spectrum distribution resources when taking sample of clock signal, and optimize the obtained clock signal sample. The zero-crossing comparators are mainly used to perform zero crossing comparison on the original MZI electrical signal and the phase-shifted MZI electrical signal to convert them into digital signals, and zero points of the MZI signal are uniformly distributed on a frequency domain. Therefore, rising edges or falling edges of the digital signals generated after zero crossing comparison are also uniformly distributed on a frequency domain. The exclusive-OR gate is mainly used to merge two digital clock signals to generate two clock signals in a free spectral range (FSR). In this way, a maximum imaging depth of OCT is increased without increasing the FSR, and jitter generated by the optical signal is reduced as well. In addition, because a sweep laser always has some idle time between two adjacent times of scanning, the optical clock signal needs to fill in some fake clock signals in the blank by using an OR gate to ensure that a high-speed analog-digital capture cards can work normally. The OR gate implements a function of merging real optical clock signals and fake clock signals. The optical clock signal output module is mainly used to transport the merged actual optical clock signals and real clock signals to the data collection module. By using the optical clock module in the OCT endoscopic scanning and imaging system, the demand of a data collection and processing system can be reduced, the collection of redundant information is reduced, and the burden of a storage system is relieved, so as to improve the integration level of the whole OCT system and to reduce the cost of the system. In addition, the signal-to-noise of an image signal can also be improved, and the attenuation of the detection sensitivity is reduced, so as to improve the definition of the image.

Preferably, a method for processing an OCT signals in an OCT endoscopic scanning and imaging system by using a General Purpose Graphics Processing Unit (GPGPU) includes four steps: (1) data collection; (2) data transmission; (3) data processing; and (4) transferring the data to an image database.

(1) Data collection: FD-OCT original data is obtained by using an external collection device in the present invention.

(2) Data transmission: the FD-OCT original data obtained in the data collection step is placed in a computer system or an embedded system memory. The data is stored in a system memory in frame as a unit. After a certain condition is satisfied (for example, data accumulates to one frame or multiple frames), the data can be transmitted to a device memory of the GPGPU by using a data bus (such as PCI Express). Because a transmission speed of the bus is relatively slow, at the same time of data transmission, the GPGPU performs parallel processing on the OCT original data which is previously transmitted to the device memory. The method has an efficient parallel signal processing capability, can implement real-time digital signal processing, greatly improves transmission efficiency and saves bus resources.

(3) Data processing: the GPGPU processes the digital signal processing for three steps: one-dimensional digital re-sampling, one-dimensional Fast Fourier Transform (FFT), and amplitude calculation and normalization. In the one-dimensional digital re-sampling step, a quick one-dimensional cubic interpolation is implemented once by linear texture lookup twice to improve the accuracy of re-sampling.

(4) Transferring the data to an image database: the processed data is placed in a memory of the image database; the image database can directly call the data which does not need to be transmitted by using a bus, thereby greatly improving the transmission efficiency and saving bus resources, providing an efficient parallel signal processing capability, implementing real-time digital signal processing, providing high transplantability, improving the flexibility of software display because of seamless combination with the popular image database (for example, post-processing can be performed on the image by the GPGPU), and implementing relatively low cost of hardware and software development.

Another objective of the present invention is to provide a non-surgical imaging method for an OCT endoscopic imaging system as defined in claim 9.

## BRIEF DESCRIPTION OF THE DRAWINGS

FIG. 1 illustrates a physical part diagram of the OCT microprobe according to the present invention;

FIG. 2a and FIG. 2b illustrates enlarged sectional views of key parts of the OCT microprobe according to the present invention;

FIG. 3 illustrates a schematic diagram of an OCT endoscopic scanning and imaging system including an OCT microprobe according to the present invention;

FIG. 4 illustrates a schematic structural diagram of a balloon catheter according to the present invention;

FIG. 5 illustrates a balloon added into a film sleeve;

FIG. 6 illustrates an imaging effect when an eccentricity between an inner tube and a balloon is approximately 500 micrometers;

FIG. 7 illustrates an imaging effect when an eccentricity between an inner tube and a balloon is less than approximately 500 micrometers;

FIG. 8 illustrates an imaging effect when an eccentricity between an inner tube and a balloon is greater than approximately 500 micrometers;

FIG. 9 illustrates a scanning result of concentricity of a product on the market.

FIG. 10 illustrates a schematic structural diagram of an automatic inflation/deflation device according to the present invention;

FIG. 11 illustrates a working flowchart of an automatic inflation/deflation device according to the present invention;

FIG. 12 illustrates a schematic diagram of an optical clock module according to the present invention;

FIG. 13 illustrates a schematic diagram of a process of generating an optical clock signal according to the present invention;

FIG. 14 illustrates a schematic diagram of an OCT endoscopic scanning and imaging system including an optical clock module according to the present invention;

FIG. 15 illustrates processing steps of an FD-OCT signal according to the present invention;

FIG. 16 illustrates a schematic diagram of parallel occurrence of GPGPU data transmission and signal processing according to the present invention;

FIG. 17 illustrates a schematic structural diagram of overall implementation according to the present invention;

FIG. 18 illustrates a curved-line relationship diagram of a working distance, a gluing distance, and a transverse resolution of an OCT microprobe in the present invention;

FIG. 19 illustrates a scanning diagram of an esophagus of a healthy animal according to the present invention;

FIG. 20 illustrates a partial enlarged view of a scanning diagram of an esophagus of a healthy animal according to the present invention; and

FIG. 21 illustrates a 3D image of an esophagus of a healthy animal according to the present invention.

## DESCRIPTIONS FOR REFERENCE NUMERALS

1, single-mode fiber, 2, spring tube, 3, glass rod, 4, grin lens, 5, reflector, 6, slotted stainless steel tube, 7, support stainless steel tube, 8, soft head, 9, inner tube, 10, balloon, 11, double-cavity tube, 12, handle, 13, ventilation interface, 14, host interface, 15, film sleeve,

81, scaly epithelial (SE) layer, 82, lamina propria (LP) layer, 83, muscularis mucosa (MM), 84, submucosa (SM), 85, muscularis propria (MP) layer,

91, MZI electrical signal on which 90° phase shift is performed after passing through a wideband 90° phase shifter; 92, MZI electrical signal on which phase shift is not performed, 93, digital signal generated after the signal 91 performs zero crossing comparison, 94, digital signal generated after the signal 92 performs zero crossing comparison, 95, fake clock signal, 96, signal generated after the digital signals 93 and 94 are merged by using an exclusive-OR gate,

101, air pump, 102, inflation solenoid valve, 103, pressure sensor, 104, throttle valve, 105, pressure sensor, 106, anti-explosion pressure sensor, 107, deflation solenoid valve, 108, mechanical pressure switch.

## DETAILED DESCRIPTION OF THE PREFERRED EMBODIMENTS

The technical solutions of the present invention are described in detail below with reference to the accompanying drawings.

Embodiment 1

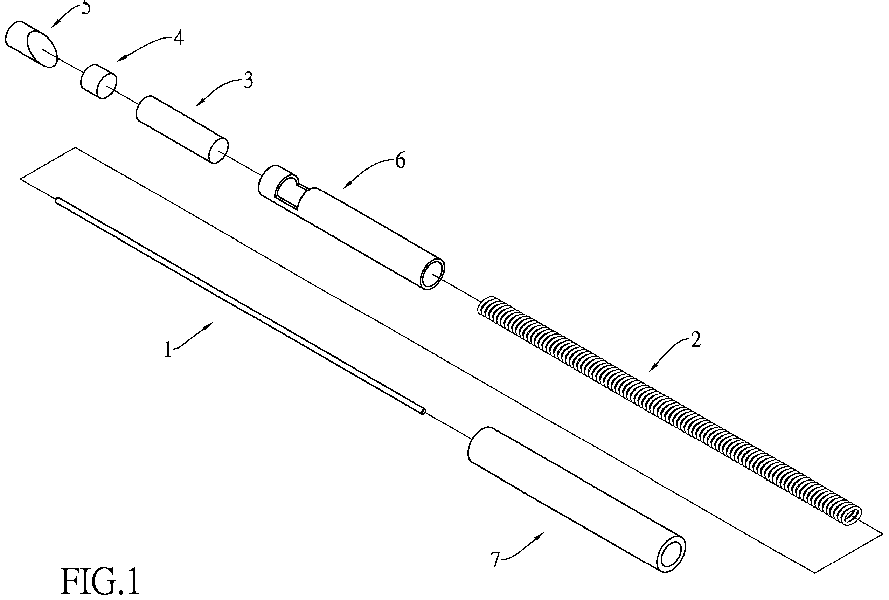

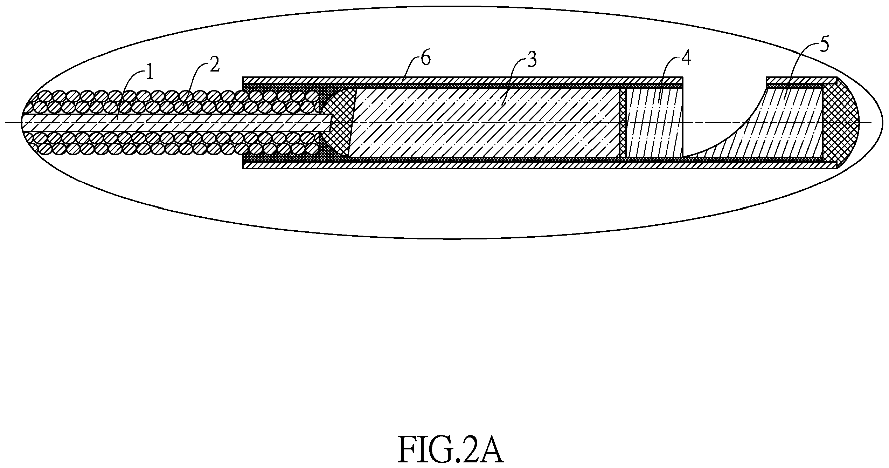

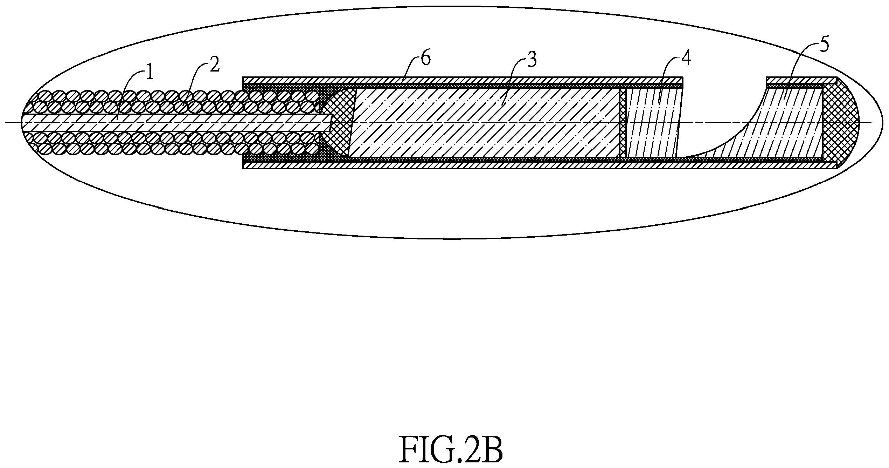

A microprobe in an optical coherence (OCT) tomography scanning system shown as FIG. 1, FIG. 2a and FIG. 2b: a single-mode fiber 1 is sleeved in a spring tube 2. The spring tube 2 can provide an enough torsion force, so that a probe of a particular length keeps synchronization between a remote end and a near end during rotation. The spring tube 2 reduces resistance during rotation of the probe while effectively protecting the fragile fiber. One end of a glass rod 3 is surface-glued to a grin lens 4 at a zero angle, and the other end is obliquely combined with the single-mode fiber 1. A working distance of an OCT probe can be changed by changing gluing distances of two end surfaces of the glass rod 3 and the single-mode fiber 1, to achieve a working distance required by expectation, thereby improving the numerical aperture and the transverse resolution of the OCT probe. A reflector 5 is a cylindrical-surface reflector and is encapsulated in a slotted stainless steel tube 6. In addition, a reflective surface of the reflector 5 faces a slotted opening of the slotted stainless steel tube 6, so as to alleviate the impact of light scattering of a light source passing through a cylindrical inner tube on imaging.

As shown in FIG. 1, FIG. 2a, and FIG. 2b, an OCT microprobe includes: a single-mode fiber 1, a spring tube 2, a glass rod 3, a grin lens 4, a reflector 5, a slotted stainless steel tube 6, and a support stainless steel tube 7. End surfaces of these optical elements are glued by using optical adhesives. Specifically, the reflector 5 is disposed into the slotted stainless steel tube 6, then is placed on working equipment for A/B adhesive dispensing, and is placed under a microscope for UV adhesive dispensing to assemble the glass rod 3 and the grin lens 4. The single-mode fiber 1 is inserted into the spring tube 2, and the assembled glass rod 3 and grin lens 4, the single-mode fiber 1, and the spring tube 2 are assembled by means of UV adhesive dispensing. At last, an assembled spring tube assembly is assembled into the slotted stainless steel tube 6, and gaps on edges are filled by A/B adhesive.

A stainless steel spring tube 2 (coated with a PTFE film) is sleeved outside the single-mode fiber 1 to reduce resistance when the probe rotates while effectively protecting a fragile fiber, so that the microprobe performs scanning more steadily and smoothly. A main function of the support stainless steel tube 7 is supporting when the OCT probe performs scanning, so that the entire probe is steadier when performing rotary scanning. A slot of the slotted stainless steel tube 6 can enable a light beam to be irradiated to a probed sample through the slot.

An antireflective film can reduce reflection of light rays between optical surfaces and enhance light transmission performance, so as to lower the impact of reflective light of the optical surfaces on signal light. Therefore, in this embodiment, by changing the length of the grin lens, a working distance of the OCT probe can be changed, and an optimal transverse resolution can be obtained under a condition of a specified working distance. An antireflective film is coated on a surface, in contact with air, of the grin lens 4. In addition, the surface, in contact with air, of the grin lens 4 can be processed into a 4° to 8° inclined surface, which can further weaken an interference signal resulted from the light passing through the surface. FIG. 2a differs from FIG. 2b in: whether the grin lens 4 has an inclination angle of an exit surface. FIG. 2b shows the inclination angle of the exit surface, which is 4° to 8°. This design minimizes the quantity of useless optical signals reflected from the surface, thereby improving the imaging quality of the OCT probe. In addition, one end of the glass rod 3 is surface-glued to the grin lens 4 at a zero angle, thereby improving the sensitivity and the resolution of the microprobe. The other end of the glass rod 3 is inclined relative to a glued surface of the single-mode fiber 1 by an angle. In this embodiment, the angle of inclination of the glued surface can be 8°. Inclination of the glued surface effectively reduces interference of the reflective light on the signal light. The glass rod 3 is designed and used to increase a numerical aperture of the OCT probe, thereby improving the transverse resolution. In addition, the working distance of the OCT probe can be changed by changing gluing distances of two end surfaces, to achieve a working distance required by expectation. The reflector 5 is installed at an angle of 45°, so that incident light rays are perpendicular to reflective light rays, causing light ray interference. In this embodiment, a 40° reflector 5 is installed on a front end of the microprobe, and the reflector 5 is encapsulated in the slotted stainless steel tube 6, and the reflective surface faces towards the slotted opening of the slotted stainless steel tube 6. In addition, to alleviate the impact of light scattering of the light source passing through the cylindrical inner tube on imaging, the reflector herein in this embodiment is designed as a cylindrical-surface reflector according to the inner and outer diameters of the cylindrical inner tube and the refractive index of the inner tube material. The reflector can change forward light rays into lateral rays and the reflector is a cylindrical-surface reflector, which can change light scattering impact caused by a protective casing outside the OCT probe on a focused light beam.

Embodiment 2

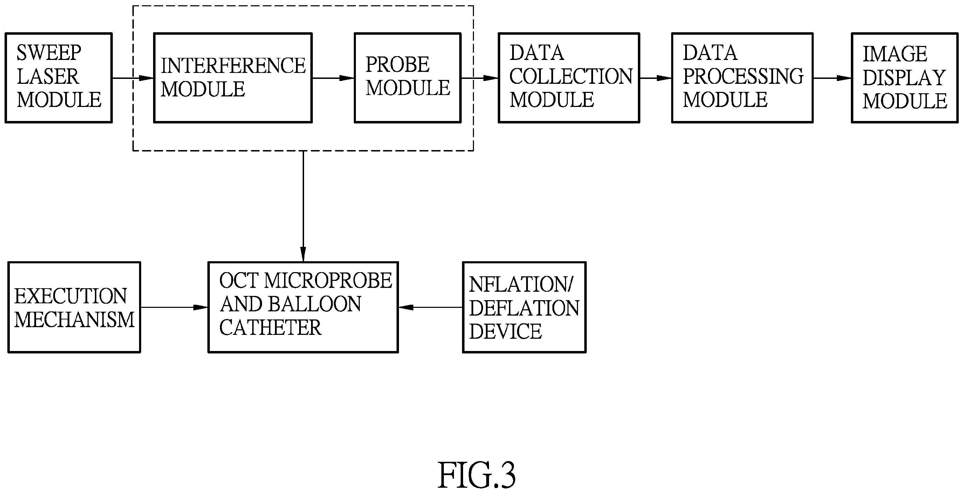

As shown in FIG. 3, an OCT endoscopic scanning and imaging system includes a sweep laser module, an interference module, a probe module, a data collection module, a data processing module, an image display module, an execution mechanism, a balloon catheter, an OCT microprobe, and the inflation/deflation device shown in FIG. 1 and Fig. 2.

The sweep laser module includes a high-speed sweep laser, a fiber isolator, and a fiber coupler. An optical signal output from the sweep laser is isolated from a subsequent optical path, to prevent an optical signal returned by the subsequent optical path from interfering with normal operation of the laser. The interference module can use a fiber-type Mach-Zehnder interferometer (MZI) or fiber-type Michelson interferometer structure. The MZI is mainly formed by two fiber couplers, two fiber circulators, and two fiber polarization controllers. A first fiber coupler is usually an asymmetric fiber coupler, and outputs most of the laser to a microprobe of a sample arm. The two fiber circulator are respectively arranged in the reference arm and the sample arm to collect optical signals reflected or scattered back from the two arms. A second fiber coupler can be a symmetric $2\times2$ fiber coupler (that is, the split ratio is 50/50) to generate an optical interference signal and reduce a direct-current common-mode signal. The fiber polarization controllers are symmetrically placed in the reference arm and the sample arm, and are used to adjust polarization states of the two arms to obtain an optimal optical interference signal. The Michelson interferometer structure is formed by a symmetric $2 \times 2$ fiber coupler, a fiber circulator, and two optical polarization controllers. The sweep laser first passes through the fiber circulator and then enters the fiber coupler. Optical signals reflected or scattered from the reference arm and the sample arm pass through a same fiber coupler and generate an interference signal. The fiber polarization controllers are symmetrically placed in the reference arm and the sample arm and are used to adjust polarization states of the two arms to obtain an optimal optical interference signal. The advantages of the MZI lie in a symmetric structure, simple dispersion management, and high detection sensitivity. The advantages of the Michelson interferometer lie in a simple structure and no introduction of polarized mode dispersion (PMD). The two interferometers are common in that an optical path difference between two arms determines a free spectral range (FSR) in which a clock signal is generated, and finally determines a maximum imaging depth of an OCT image. The probe module can be a high-speed balanced photodetector and is mainly used to convert an interference optical signal output from the interference module into an electrical signal. The data collection module is a high-speed analog-digital collection card and is mainly used to convert an analog electrical signal into a digital electrical signal and provide the digital signal to the data processing module to process the digital signal. The data processing module is a chip (such as a CPU, a GPGPU, a DSP, or an FPGA) having a digital signal processing capability and is mainly used to process an original signal and convert the original signal into a final image signal. The image display module is mainly used to display the image signal and is responsible for post-processing and measurement of the image. The execution mechanism is formed by a fiber rotation connector, a motor, and a motorized translation stage and is mainly used to drive the OCT microprobe to perform mechanical and spiral scanning to obtain an OCT image. The OCT microprobe is mainly used to enter an internal organ of a human body to transmit sweep laser and collect an optical signal scattered backwards from tissues. The balloon catheter is used to expand tracts of internal organs of a human body, eliminate folds, and stabilize the OCT microprobe at the center of the balloon. The inflation/deflation device is mainly used to expand the balloon catheter.

Embodiment 3

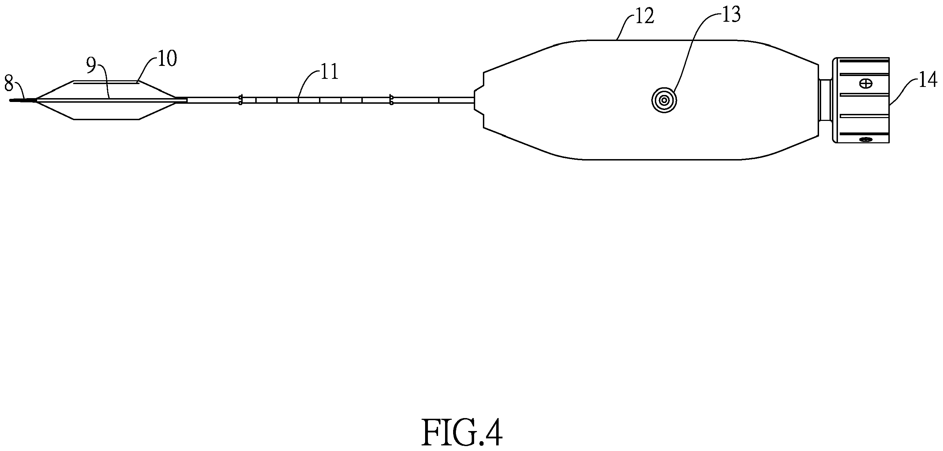

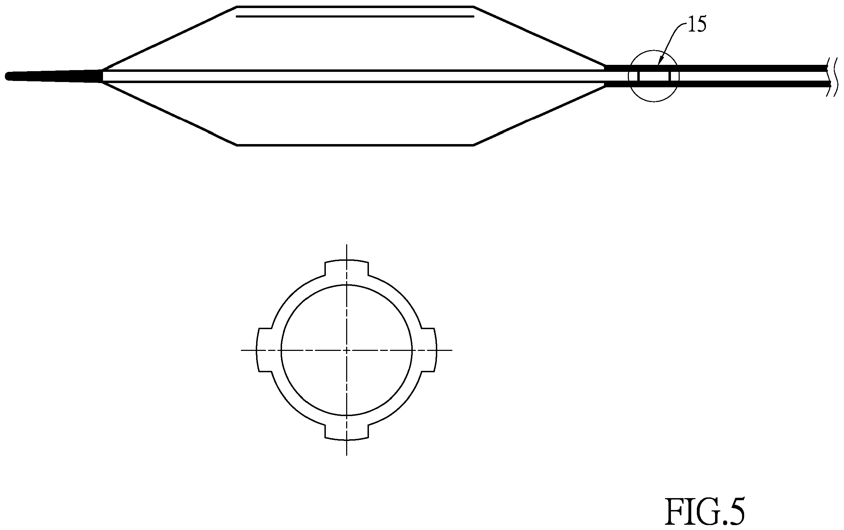

An OCT endoscopic scanning and imaging system is similar to Embodiment 2. The difference between the OCT endoscopic scanning and imaging system and Embodiment 2 is as follows. As shown in FIG. 4, the balloon catheter includes: a handle 12, where one interface of the handle 12 is a host interface 14, and the other interface is a ventilation interface 13; a double-cavity tube 11, allowing an OCT optical probe to pass through; a balloon 10, where a front end of the balloon 10 is blocked, scales are provided on the balloon, a solid welded structure is blocked on the front end, a blocking socket thereof is disposed within an inner tube, when a device moves within a cavity, the cavity rubs with the inner tube and the balloon and is not in contact with the blocking soft head, and even if the cavity is in contact with the soft head, a friction force tends to press the soft head into the inner tube; an inner tube 9, where the concentricity between the inner tube 9 and the balloon 10 is an offset of no more than 500 micrometers under three rated working atmospheric pressures; a film sleeve 15, where as shown in FIG. 5, the film sleeve is located at a junction between the balloon and a double-cavity tube and controls the inner tube to float within a lumen, so as to ensure that the inner tube does not offset from the center of the balloon, and resolve a problem of being eccentric; and a soft head 8, which is a solid structure. One end of the double-cavity tube 11 is connected to the handle 12, and the other end is connected to one end of the inner tube 9 and one end of the balloon 10. The other end of the balloon 10 and the other end of the inner tube 9 are connected to the soft head 8. The double-cavity tube 11 is connected to the handle 12 by using a UV adhesive, and all the other parts are connected in a welded manner.

The material of the handle 12 is polycarbonate, the materials of the double-cavity tube 11 and the soft head 8 are block polyether amide, and the materials of the balloon 10 and the inner tube 9 are nylon and a modified polymer thereof.

Ink printing scales whose line width is less than or equal to 0.1 mm are provided on the balloon 10, so that a scanning direction of the probe can be recognized. This does not affect scanning judgment of a normal image and can also recognize a scanning position on a display screen. Scales are printed on the double-cavity tube 11. Therefore, a doctor can determine a scanning position. A conventional balloon catheter needs to be supported and guided by a guide wire. The diameter of the guide wire is usually 0.018 in, 0.035 in, 0.014 in, and 0.038 in. The double-cavity tube 11 used in the OCT balloon catheter in this embodiment can pass through a 0.055-in OCT optical probe. When optical imaging is performed, a probe light beam emitted out by the probe needs to pass through a wall of the inner wall to reach a probed object. In addition, during imaging, the OCT probe also needs to rotate within the inner tube, so as to comprehensively probe the probed object. In this process, if the wall of the inner tube is excessively thick, because probe light attenuates when passing through the wall of the inner tube, energy loss of probe light is caused, and consequently, imaging is unclear. If the wall of the inner tube is excessively thin, because the OCT probe rotates at a high speed within the inner tube, the excessively thin wall of the inner tube causes a low speed of the probe, and deformation of the inner tube, and consequently, imaging is non-uniform. Therefore, this application defines the inner diameter and the outer diameter of the inner tube to determine the thickness of the inner tube. In this embodiment, the inner diameter of the inner tube 9 is 1.4 mm, and the outer diameter is 1.65 mm, which are exclusively designed for the OCT probe. The thickness of the inner tube is most appropriate for OCT probe imaging, can ensure that light energy attenuation under this thickness is relatively small, and also ensure strength of the inner tube. A problem of non-uniform and unclear imaging and unclear imaging caused by a low speed of the probe and deformation of the inner tube does not occur.

The length of the inner tube is determined according to the length of the balloon, and the length thereof is less than that of the balloon. When the balloon is welded with the soft head, the balloon is pushed downwards by a distance, so as to be aligned with and fixed to the inner tube for welding, so that the balloon has an elongation allowance when the balloon is filled, so as to match stretching of the inner tube and keep concentric.

When optical imaging is performed, a tract in which a probed object is located needs to be expanded by the balloon, so as to make imaging complete. If an air pressure is relatively low, folds are formed on the surface of the balloon, and this necessarily affects imaging effects. However, if the air pressure is excessively high, it may damage and destroy the probed object. Therefore, the applicant preferably adopts an option of three atmospheric pressures to ensure that the device does not hurt the probed object during use and the balloon can be completely filled without wrinkles. In addition, the option can also define or limit the length of the balloon and the length of the inner tube. The process of the balloon of this application can ensure that the concentricity deviation between the inner tube and the balloon is no more than 500 micrometers under three atmospheric pressures, so as to facilitate optical imaging.

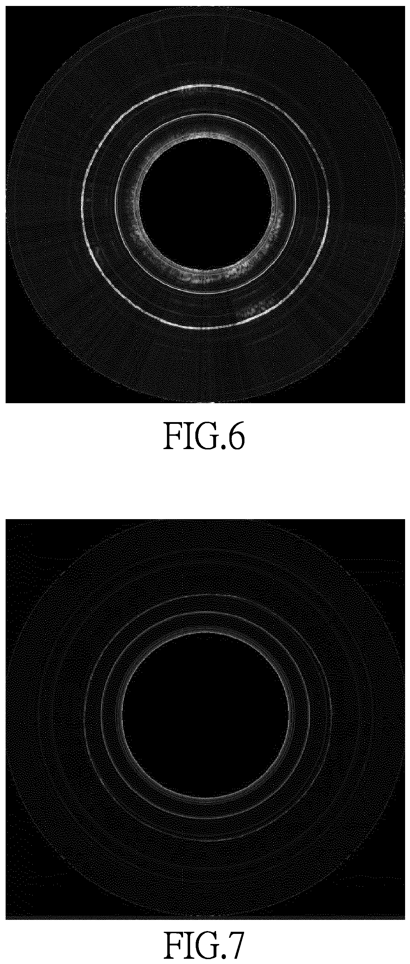

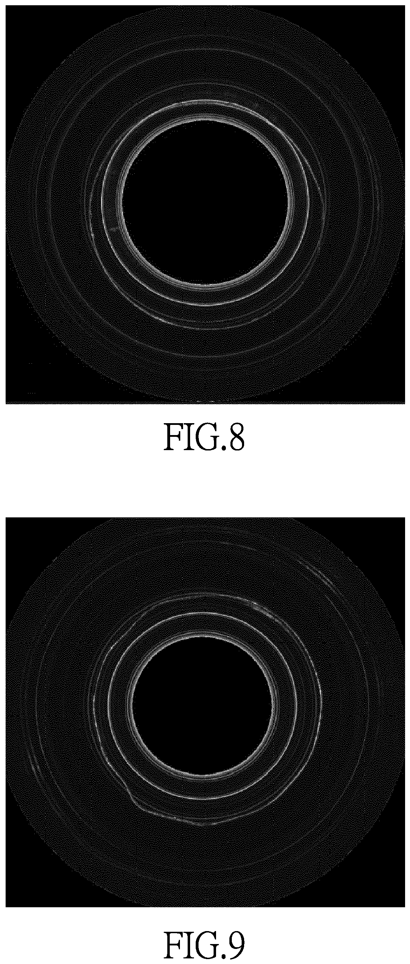

If the concentricity deviation between the inner tube and the balloon is no more than 500 micrometers, an imaging effect is relatively good. If the offset between the inner tube and the balloon is more than 500 micrometers, a generated image may be incompletely displayed. FIG. 6 shows a standard circle whose eccentricity is approximately 500 micrometers. FIG. 7 and FIG. 8 are respectively images that are scanned when the eccentricity of the inner tube and the balloon is less than 500 micrometers and when the eccentricity exceeds 500 micrometers. It can be seen from the foregoing two figures that the eccentricity less than 500 micrometers can meet a requirement of complete imaging. Upon comparison, a general product on the market does not control the index and cannot meet a requirement that the concentricity between the inner tube and the balloon is an offset of no more than 500 micrometers under three atmospheric pressures. A product on the market is selected for contrast, and an imaging effect is shown in FIG. 9. Imaging of an area on the upper right corner is incomplete and an actual scanning requirement cannot be met.

Embodiment 4

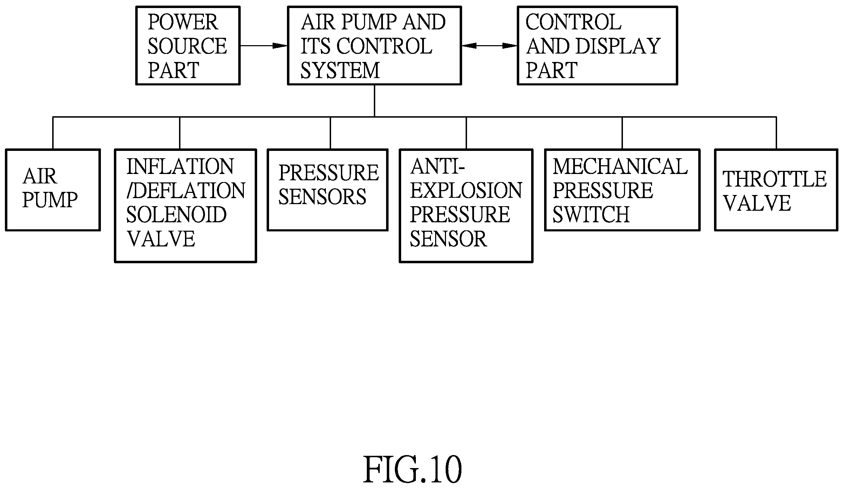

An optical coherence tomography scanning system similar with embodiment 2 and 3, the difference is that the inflation/deflation device is an automatic inflation/deflation device. As shown by FIG. 10, the automatic inflation/deflation device includes a power source part, a control and display part, and an air pump and its control system. The air pump and its control system include: the air pump, an inflation solenoid valve, a deflation solenoid valve, a throttle valve, pressure sensors, an anti-explosion pressure sensor, and a mechanical pressure switch.

There are multiple pressure sensors in this application. At least one of the pressure sensors is connected to a balloon. At least one of the pressure sensors is disposed between a throttle valve and an inflation solenoid valve and a deflation solenoid valve. The pressure sensor functions to monitor, in real time, a pipeline air pressure and a balloon air pressure in an inflation and deflation process. The pressure sensors include two pressure sensors 103 and 105 and form a pressure monitoring system of the inflation and deflation process together with multiple solenoid valve components in this application. The specific structure is: the inflation solenoid valve 102 and the deflation solenoid valve 107 are provided between the air pump 101 and the pressure sensor 103; the pressure sensor 103 is separated from the pressure sensor 105 by the throttle valve 104; the pressure sensor 105 is connected to the balloon. The following three effects can be achieved by means of this structural setting:

(1) Two pressure sensors 103 and 105 are disposed and are separated from each other by the throttle valve 104. Then, the pressure sensor 105 is directly connected to the balloon, and obtained data is more accurate. The pressure sensor 103 not only can monitor the pipeline air pressure for checking an air pressure of the pressure sensor 105, but also can implement a function of early warning and monitoring of inflation and deflation pressures of the air pump after the inflation solenoid valve 102 and the deflation solenoid valve 107 are opened and before the throttle valve 104 is opened in the inflation and deflation process. The pressure sensor 103 can close the solenoid valves in time by means of automatic control if there is a problem about the pressures, so as to prevent excessively strong inflation and deflation pressures from causing unnecessary damages to the balloon or even body cavities of a patient, thereby greatly enhancing the security of the system.

(2) The pressure sensors 103 and 104 are both connected to the air pump and the outside by using the solenoid valves, and interference of the air pump and an outside air pressure to pressure monitoring of the pressure sensors is isolated by using the solenoid valves.

(3) Double monitoring is performed on the balloon and the pipeline air pressure. Even if a single sensor has a fault, the system can still normally work, enhancing the stability of the system.

The pressure monitoring system of this application further includes an anti-explosion pressure sensor connected to the balloon. The anti-explosion pressure sensor functions to monitor the air pressure in real time in the inflation and deflation process. Data monitored by the anti-explosion pressure sensor is independent of data monitored by the pressure sensors. When the monitored data exceeds a preset air pressure upper limit value, a pressure relief protection mechanism is started. If the monitored data keeps unchanged for a long time, the anti-explosion pressure sensor prevents continued pressurization of the air pump. The pressure monitoring system of this application is divided into the pressure sensors and the anti-explosion pressure sensor. Multi-monitoring on the air pressure is implemented by means of disposing multiple sensors, thereby enhancing the stability of the system. In addition, different sensors are disposed at different positions and can perform mutual checking and verification. The solenoid valves are provided between sensors for isolation, so as to avoid interference of an inflation/deflation device and a pressure relief device on pressure measurement, thereby improving the accuracy of pressure measurement. At last, the pressure monitoring system formed by the multiple pressure sensors together with the solenoid valves can prevent excessively strong inflation and deflation pressures from causing unnecessary damages to the balloon or even body cavities of a patient with the support of an automatic control function, thereby enhancing the security of the system.

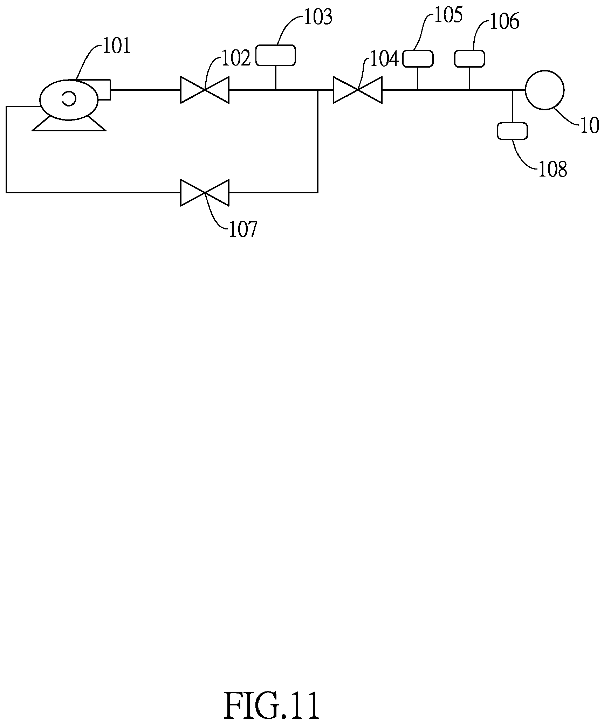

As shown in FIG. 11, a process for using the automatic inflation/deflation device includes:

1) Inflation process: first, a user sets parameters such as an air pressure and an inflation time of the balloon 10, and issues an inflation command; the control system reads data of the pressure sensors 103 and 105; if the data is less than the air pressure set by the user, the air pump 101 is started, and the inflation solenoid valve 102 and the throttle valve 104 are opened; feedback values of the pressure sensors 103 and 105 are read in real time in the inflation process; the balloon 10 is inflated until the set air pressure value is reached, and then the air pump 101, the throttle valve 104, and the inflation solenoid valve 102 are closed.

(2) Deflation process: first, the user sets parameters such as the air pressure and a deflation time of the balloon 10; the control system reads data of the pressure sensor 105; if the data is greater than the air pressure set by the user, the air pump 101 is started and the deflation solenoid valve 107 and the throttle valve 104 are opened; a feedback value of the pressure sensor 105 is read in real time in the deflation process; air is extracted from the balloon 10 until the set air pressure value is reached, and then the air pump 101, the throttle valve 104, and the deflation solenoid valve 107 are closed.

In the inflation and deflation process, the system monitors a feedback value of the anti-explosion pressure sensor 106 in real time; if the feedback value exceeds an air pressure upper limit value set by the user, software program protection is immediately performed. The air pump 101 is closed, and an alarm is given. The mechanical pressure switch 108 is hardware protection. If the feedback value exceeds the set value, the switch is opened, and pressure relief is performed for protection.

The pressure relief protection mechanism is implemented by guiding an entire inflation/deflation device system by means of an automatic control function, is triggered by monitoring by using the anti-explosion pressure sensor, and is implemented in three ways. First, software protection: when the anti-explosion pressure sensor monitors that the pressure exceeds a limit, an air pump inflation function is disabled and the inflation solenoid valve is also closed; if necessary, the throttle valve and the deflation solenoid valve are opened, and the air pump actively exhausts air and relieves the pressure. In the process, the pressure sensor cooperates to perform pressure monitoring. After the pressure is normal, the solenoid valve and the air pump are closed to end software-protected pressure relief, to prevent excessive pressure relief from causing dangers. Secondly, hardware protection: after the anti-explosion pressure switch monitors that the pressure exceeds the limit, the mechanical switch is automatically opened for pressure relief protection. Thirdly, pressure relief valve protection: when the device cannot normally run, a switch on the pressure relief may further be opened to perform manual pressure relief. In this application, three protection ways: software protection, hardware protection, and pressure relief valve protection are provided for the over-pressure risk of the system in cooperation with the anti-explosion pressure sensor, thereby enhancing the security and the stability of the system and improving the accuracy of the pressure relief system.

In addition, the automatic inflation/deflation device of this application also has the following advantages:

1. A user can set needed parameters, such a pressure value and an inflation time, on a control and display module at anytime, and uploads relevant parameters in real time to a data system. The system immediately performs monitoring and operation according to new parameters, and can make in-time responses to various accidents during the operation process, thereby enhancing the system security. In addition, accurate air pressure control enables relatively high consistency of the shape of the inflated balloon and higher relevance of obtaining image data for multiple times from a scanned object, facilitating comparison.

2. If the air pressure of the air pump does not change or slightly changes after long-time work of the air pump, the balloon may be broken or air may be leaked from the balloon. If the air pump continues to inflate or deflate, it may cause injury to a body cavity of a patient may. Therefore, if the pressure monitoring system detects that the air pressure does not change or slightly changes when the air pump inflates or deflates for a long time, the pressure monitoring system automatically starts a protection mechanism and turns off the air pump, thereby enhancing the system security.

3. In this application, the solenoid valves can be quickly opened and closed in an automatic control manner, thereby reducing interference of inflation and deflation on the monitoring system and enhancing the accuracy of the monitoring system.

4. In this application, inflation and air exhaustion are performed in two different channels, and two processes of inflation and deflation can be switched quickly. Especially in the pressure relief protection process, fast air exhaustion can well prevent over-inflation from damaging the balloon and a body cavity of a patient, thereby enhancing the system security.

5. In this application, an automatic inflation and deflation air pump is used, and an inflation pressure range thereof is relatively large. The pressure range is usually controlled in a range from one to five atmospheric pressures according to the body cavity environment used in this application. An air pressure range that can be provided by manual pressure supply is far less than the pressure range in this application.

Embodiment 5

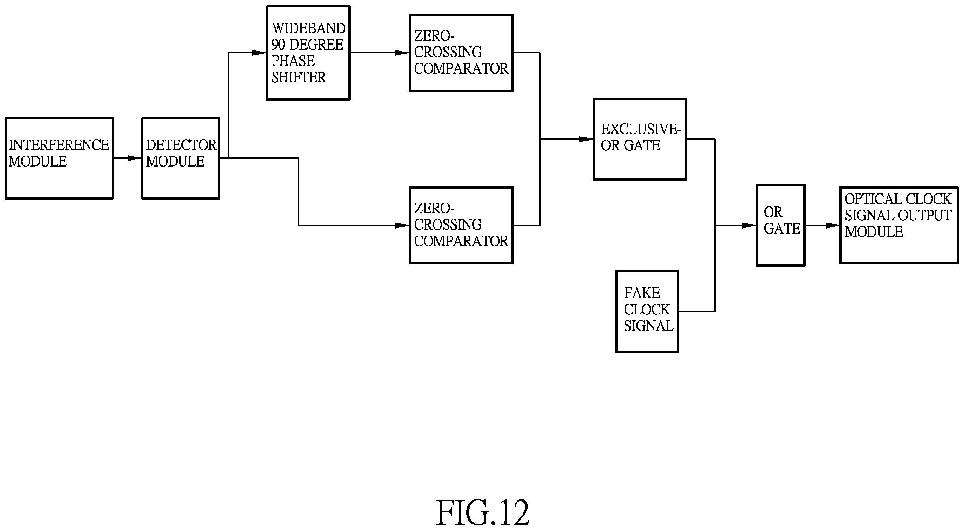

FIG. 12 is an optical clock module using in an OCT endoscopic imaging system, comprising an interference module, a detector module and an optical clock conversion circuit module, the optical clock conversion circuit module includes a wideband 90-degree phase shifter, two zero-crossing comparators, a circuit composed of exclusive-OR gate and OR gate and an optical clock signal output module. The interference module adopts an all-fiber Mach-Zehnder Interferometer (MZI) structure which is mainly consists of two fiber couplers. A second coupler is a symmetric 2×2 fiber coupler. First of all, light is divided into two paths at the first fiber coupler, these two paths of light respectively pass through a first fiber and a second fiber that have a fixed optical path difference, interference occurs at the second fiber coupler. The probe module, composed of a high-speed balanced photodetector, is mainly used to convert an interference optical signal output from the interference module into an electrical signal. A part of the MZI electrical signal converted by the detector module is transmitted to the wideband 90-degree phase shifter and the other part is transferred to the zero-crossing comparator.

The phase of the electrical signal transmitted to the wideband 90-degree phase shifter is shifted for 90 degrees. The zero-crossing comparators are mainly used for the zero-crossing comparison of the phase-shafted signal and the non-phase-shafted signal and convert them to digital signals. The exclusive-OR gate is mainly used to merge two digital clock signals to generate two clock signals in a free spectral range (FSR). In this way, a maximum imaging depth of OCT is increased without increasing the FSR, and jitter generated by the optical signal is reduced as well. In addition, because a sweep laser always has some idle time between two adjacent times of scanning, the optical clock signal needs to fill in some fake clock signals in the blank by using an OR gate to ensure that a high-speed analog-digital capture cards can work normally. The OR gate implements a function of merging real optical clock signals and fake clock signals. The optical clock signal output module is mainly used to transport the merged actual optical clock signals and fake clock signals to the data collection module.

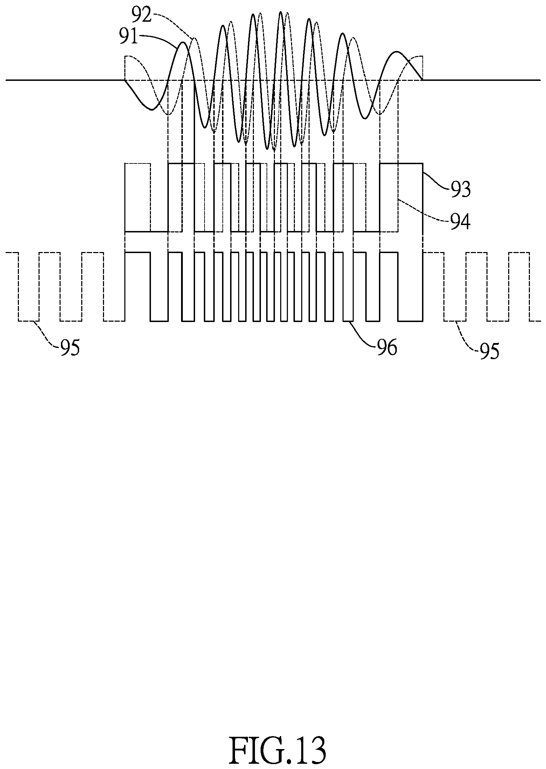

As shown in FIG. 13, reference number 91 indicates a MZI electrical signal that has been phase shifted by 90 degrees through the wideband 90 degree phase shifter; reference number 92 indicates a MZI electrical signal without phase shift; reference number 93 indicates a digital signal derived from the zero-crossing comparison of phase-shifted MZI signal 91; reference number 94 indicates a digital signal derived from the zero-crossing comparison of the MZI signal 92 without phase shift. Since the zero points of the MZI signal are uniformly distributed on a frequency domain, rising edges or falling edges of the digital signals derived from zero crossing comparisons are also uniformly distributed on the frequency domain. Reference number 96 indicates a merged signal, merged by an exclusive OR gate, of the digital signals 93 and 94 that are uniformly distributed on the frequency domain. Reference number 95 indicates a fake clock signal. The signals 95 and 96 are combined together by the OR gate to form the optical clock signal.

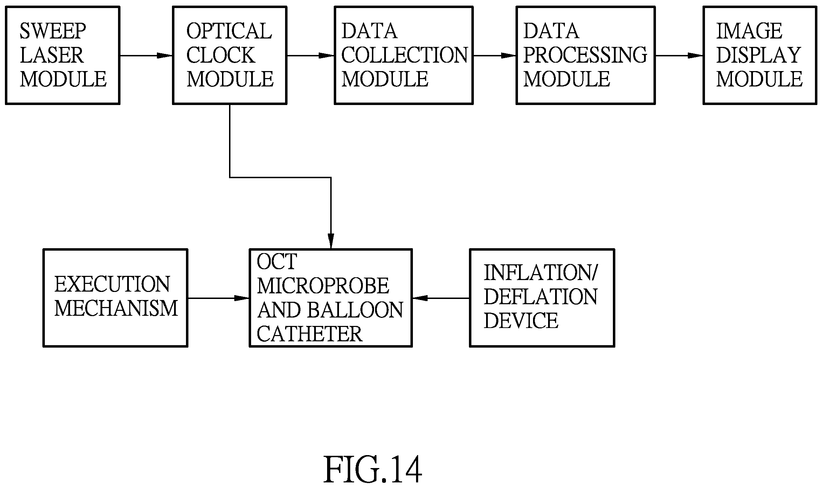

As shown in FIG. 14, an OCT endoscopic scanning and imaging system is similar with embodiments 2-4. The difference is that it comprises a sweep laser module, an optical clock module, a data collection module, a data processing module, an image display module, an actuator, an OCT microprobe, a balloon catheter, and an automatic inflation/deflation device. The optical clock module, as shown in FIG 12, comprises an interference module, a detector module and an optical clock conversion circuit module, the optical clock conversion circuit module includes a wideband 90-degree phase shifter, a zero-crossing comparator, a circuit composed of exclusive-OR gate and OR gate and an optical clock signal output module. The optical interference signal generated by the MZI is converted into an electrical signal by the balanced photodetector. The electrical signal is then converted into an optical clock signal that is uniform in the frequency domain and frequency- variable in time domain by a circuit composed of a wideband 90-degree phase shifter, zero-crossing comparators, an exclusive-OR gate. The OR gate merges the real optical clock signals and fake clock signals to ensure that a high-speed analog-digital capture cards can work normally.

The sweep laser module comprises a high-speed sweep laser, a fiber isolator, a fiber coupler, it isolates the optical signal of the sweep laser from the subsequent optical path so as to prevent the normal operation of the laser from being interfered by the optical signal of the subsequent optical path. A small part of the sweep laser is split into the optical clock module, most of the laser continue to be output. The optical clock module comprises the interference module, the probe module, and an optical clock conversion circuit module. It is mainly used to obtain an optical clock signal that is uniform in frequency domain and frequency-variable in time domain. The data collection module can be a high-speed analog-digital collection card and is mainly used to collect the original image signal based on the optical clock signal output from the optical clock module, and then the original image signal is provided to the data processing module for processing. The data processing module is a chip (such as a CPU, a GPGPU, a DSP, or an FPGA) having capability of digital signal processing and is mainly used to process an original signal and convert the original signal into a final image signal. The image display module is mainly used to display the image signal and is responsible for post-processing and measurement of the image. The execution mechanism is formed by a fiber rotation connector, a motor, and a motorized translation stage and is mainly used to drive the OCT microprobe to perform mechanical and spiral scanning to obtain an OCT image. The OCT microprobe is mainly used to enter an internal organ of a human body to transmit sweep laser and collect an optical signal scattered backwards from tissues. The balloon catheter is used to expand tracts of internal organs of a human body, eliminate folds, and stabilize the OCT microprobe at the center of the balloon. The inflation/deflation device is mainly used to expand the balloon catheter.

Embodiment 6

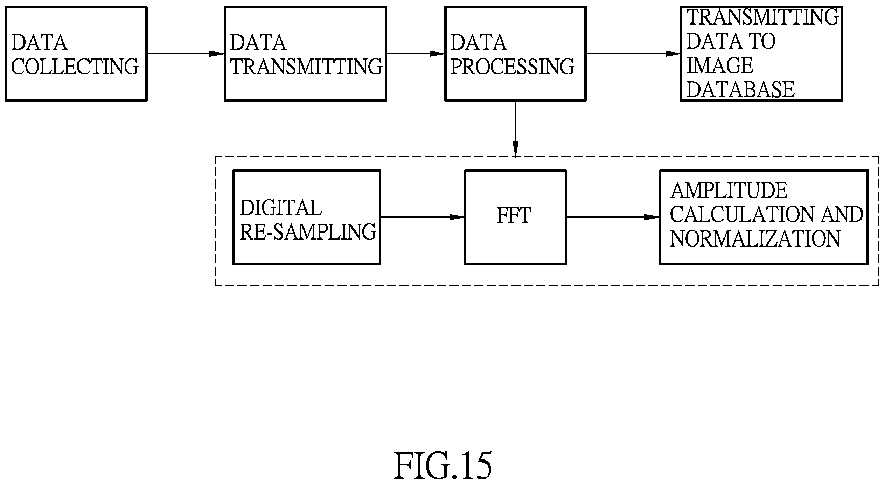

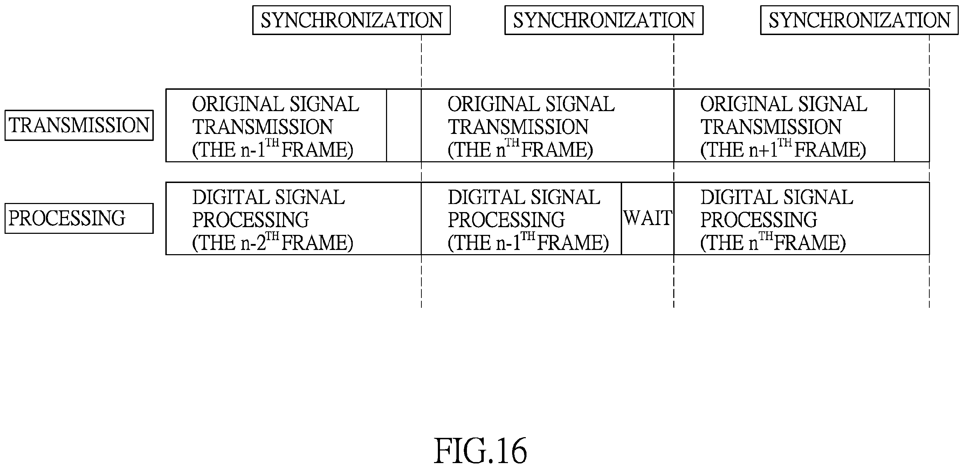

An OCT endoscopic scanning and imaging system is similar to Embodiment 2-5. The difference between embodiment and the OCT endoscopic scanning and imaging system and Embodiment 2-5s is the embodiment 6 further using a method for processing OCT signals by GPGPU as shown in FIG. 15. The method includes the steps: (1) data collecting; (2) data transmitting; (3) data processing; and (4) transmitting the data to an image database. Because a transmission speed of the bus is relatively slow, at the same time of data transmission, the GPGPU performs parallel processing on the OCT original data been previously transmitted to the device memory. The parallel processing is shown in FIG.16. The data processing comprises three steps: one-dimensional digital re-sampling, one-dimensional Fast Fourier Transform (FFT), and amplitude calculation and normalization. In the one-dimensional digital re-sampling step, a quick one-dimensional cubic interpolation is implemented once by searching for linear texture twice to improve the accuracy of re-sampling.

As shown in FIG. 15, a method for processing OCT signals using GPGPU including the steps: (1) data collecting; (2) data transmitting; (3) data processing; and (4) transmitting the data to an image database.

(1) Data collecting: FD-OCT original data is obtained by using an external collection device.
(2) Data transmitting: the FD-OCT original data obtained in the data collection step is stored in a computer system or an embedded system memory. The data is stored in a system memory in frames. When a certain condition is satisfied (for example, data have been accumulated enough to form one frame or multiple frames), the data can be transmitted to a device memory of the General Purpose Graphics Processing Unit (GPGPU) by using a data bus (such as PCI Express). Because a transmission speed of the bus is relatively slow, during the same time of data transmission, the GPGPU performs parallel processing on the OCT original data previously transmitted to the device memory.
For example, as shown in FIG. 16, when the original data of the n-th frame is transferred to the memory of the GPGPU, the original data of the (n-1)-th frame is subjected to digital signal processing in the GPGPU at the same time. A frame synchronization is performed after the end of data transmitting and data processing, that is, whichever the process of the data transmitting or the data processing completes first, there is a waiting for process of the next frame. The parallel signal transmitting/processing can effectively improve the data processing speed of the GPGPU.
(3) Data processing: As shown in FIG. 15, digital signal processing in the GPGPU comprises three steps: one-dimensional digital re-sampling, one-dimensional Fast Fourier Transform (FFT), and amplitude calculation and normalization. The digital re-sampling can be realized by the built-in texture lookup function of the Graphics Processing Unit (GPU). The built-in texture lookup function of the GPU can automatically realize the two-dimensional linear interpolation. Since the texture lookup module of the GPU has been optimized in hardware for interpolation, interpolation processing speed of the texture lookup module is faster than the general image processor, especially for OCT signal processing in the non-equidistant interpolation. By precisely setting a search point, one-dimensional linear interpolation can be realized through the built-in texture lookup function of the GPU. On the basis of this, performing texture lookup twice can quickly achieve one-dimensional cubic interpolation, so as to improve the accuracy of re-sampling. As the texture lookup module is optimized for the non-equidistant interpolation, the calculation amount is smaller and the calculation efficiency is higher than the cubic interpolation on the GPGPU. FFT can be implemented through a common commercial image processor-based numerical calculation library (such as the cuFFT library of nVidia or the OpenCL FFT library). Calculation of the amplitude and normalization can be achieved by programming an image processor. For example, using the CUDA library provided by nVidia for writing the corresponding kernel function to achieve fast traversal of two-dimensional data, amplitude calculation and normalization can be achieved.

(4) Transferring the data to an image database: the processed data is stored in a memory of the image database; the image database can directly access the data which does not need to be transmitted by using a bus, thereby greatly improving the transmission efficiency, saving bus resources, providing an efficient parallel signal processing capability, implementing real-time digital signal processing, and providing high transplantability. Because of seamless combination with the popular image database (for example, post-processing the image by using the GPGPU), the flexibility of software display is improved and hardware and software development cost can be reduced.

Embodiment 7

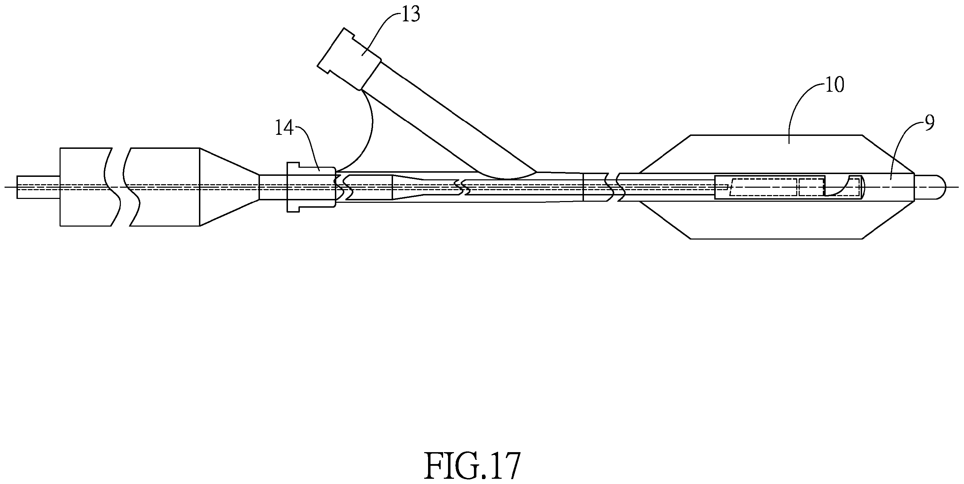

FIG. 17 illustrates a microprobe for OCT imaging scanning in a human body esophagus. The microprobe is inserted from a near-end handle cavity of a balloon into a balloon inner tube 9. A ventilation interface 13 of the balloon handle is connected to an automatic air pump (not shown in the figure). The balloon 10 is inflated to a rated air pressure to expand an esophagus. The balloon 10 and the inner tube 9 both are made of optical transparent materials having extremely high light transmission performances. The balloon 10 functions to expand the esophagus to reduce esophagus folds and keep the OCT microprobe within a working distance range thereof. The radius of expansion of the balloon 10 is approximately in a range from 8 mm to 10 mm, which is also the radius of the esophagus when the esophagus is completely expanded. Therefore, a relatively long working distance (approximately 8 mm to 10 mm) is a characteristic that the OCT microprobe needs to have. The OCT microprobe needs a relatively long grin lens when the working distance is relatively long. The relatively long grin lens is not easy to be bent and may cause inconvenience when it is used within a body cavity. The lens may be cracked when it is bent excessively, and there are safety risks. Therefore, the working distance of the grin lens is changed by disposing the glass rod, so that the grin lens becomes a multi-section structure, thereby ensuring that the microprobe has a better over-bending property, so that the entire microprobe can still enter a narrow channel when the working distance is relatively long.

The glass rod 3 is especially designed in this embodiment. It is mentioned above that the working distance of the microprobe can be changed by changing the gluing distance between the glass rod 3 and the single-mode fiber 1. The microprobe in this embodiment is used in a human body esophagus. The working distance is approximately 8 mm to 10 mm. By means of calculation and tests, the gluing distance between two surfaces of the glass rod 3 and the single-mode fiber 1 should be less than 0.3 mm. The glass rod 3 in the application not only enables the OCT probe to work within a relatively long working distance but also changes the numerical aperture and the transverse resolution of the microprobe, so as to increase the numerical aperture and improve the transverse resolution of the microprobe while changing the gluing distance between the glass rod and the single-mode fiber and increasing the working distance.

A relationship between the numerical aperture and a clear aperture of an optical element is as follows:

$$\mathrm{N.A.} = \frac{D/2}{\mathrm{W.D.}}$$

Wherein, D is the clear aperture of the optical element, W.D is the working distance, and N.A is the numerical aperture. When the working distance W.D. is fixed, the numerical aperture is in direct proportion to the clear aperture (D) of the optical element. Because of defects of the manufacturing process of the grin lens, the clear aperture is only approximately 80% of the diameter thereof. However, because the single-mode fiber is relatively thin, in an actual application, the clear aperture actually used by the grin lens directly connected to the single-mode fiber is only less than 10% of the diameter of the grin lens. To increase the clear aperture actually used by the grin lens, the glass rod is added in this application. Because light is diffusely transmitted in the glass rod, the glass rod implements a beam expansion function for light rays, thereby increasing the clear aperture actually used when the fiber passes through the grin lens.

A relationship between the resolution and the numerical aperture is as follows:

$$\Delta x \ (\text{transverse resolution}) \propto \frac{\lambda}{\mathrm{N.A.}}$$

wherein $\lambda$ is an incident light wavelength and is a fixed value. The transverse resolution $\Delta X$ is in direct proportion to the numerical aperture (N.A), that is, a larger numerical aperture indicates a higher transverse resolution (a smaller value).

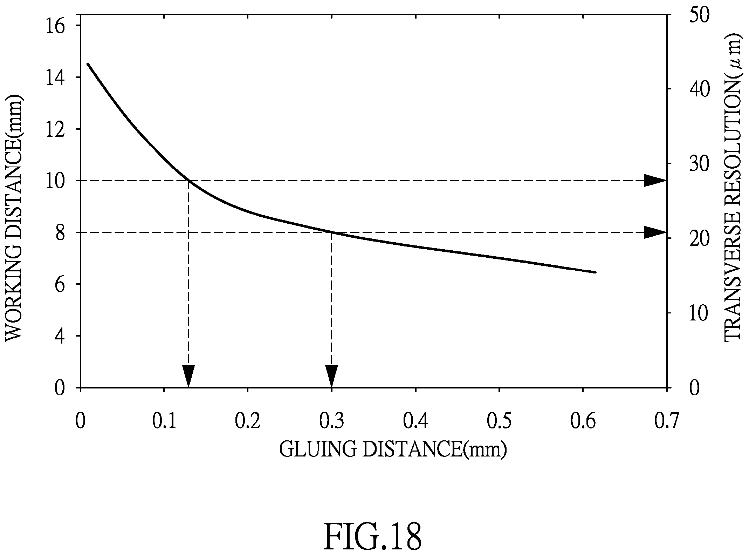

In conclusion, the use of the glass rod 3 not only increases the working distance of the microprobe, but also increases the numerical aperture of the microprobe. The increase in the numerical aperture also increases the transverse resolution. In addition, this design also greatly shortens the length of the grin lens and ensures the over-bending property of the microprobe. In this way, the entire microprobe still can directly enter a human body esophagus together with the balloon catheter by using an endoscopic pliers channel. This effective design enables the transverse resolution of the probe to reach approximately 10 to 30 micrometers, and enables the working distance to reach 8 mm to 10 mm. A relationship between the working distance and the transverse resolution of the microprobe is shown in FIG. 18. The diameter of the entire microprobe is less than 1.5 mm. If a grin lens with a diameter being 1.0 mm is used, the diameter of the entire microprobe is less than 1.3 mm. If a grin lens with a diameter being 0.7 mm is used, the diameter of the entire microprobe is less than 1.0 mm. If a grin lens with a diameter being 0.5 mm is used, the diameter of the entire microprobe is less than 0.7 mm. This application enables the OCT microprobe to be applied to a narrow space and have a relatively long working distance, a relatively large numerical aperture, and a relatively high resolution.

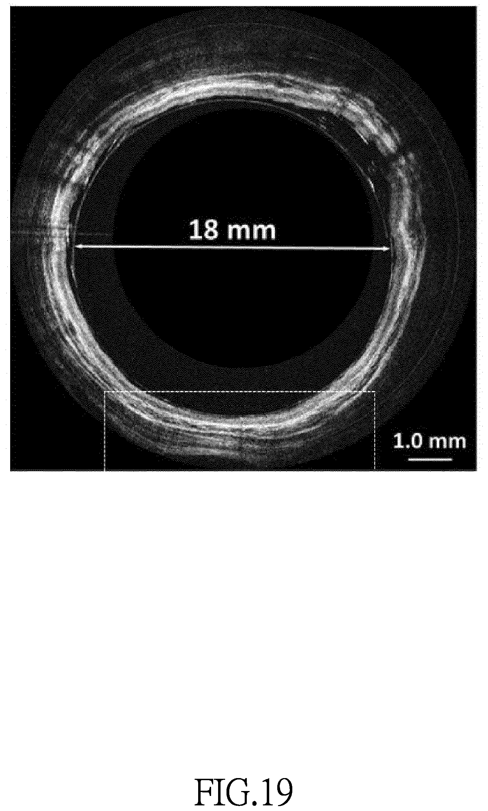

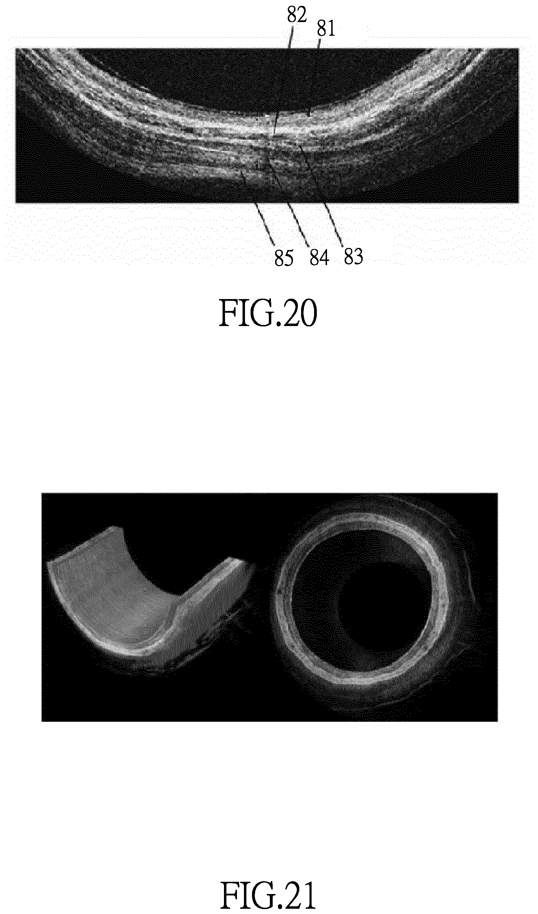

FIG. 19 shows an image of a section of an esophagus of a healthy animal, which is obtained by using an OCT endoscopic scanning and imaging system. The size of the image is 1200 transverse scanning number×4096 longitudinal scanning, a scanning speed is 0.2 cm/3s, and a scale is 1 mm. FIG. 20 is a partial enlarged view of the image of the esophagus of the healthy animal of FIG. 19. Recognizable layers include: 81: scaly epithelial (SE) layer, 82: lamina propria (LP) layer, 83: muscularis mucosa (MM), 84: submucosa (SM), and 85: muscularis propria (MP) layer. FIG. 21 is a 3D image, generated after the OCT endoscopic scanning and imaging system performs lumen surface and depth scanning and then performs software reestablishment on scanned data, of an esophagus of a healthy animal.

The foregoing descriptions are merely preferred implementations of this application and enable a person skilled in the art to understand or implement the invention of this application. Multiple modifications and combinations of these embodiments are obvious to a person skilled in the art.

**Claims**

1. An OCT microprobe comprising:

   a spring tube (2), which is capable of providing sufficient torque to keep synchronization of both a distal tip and a proximal tip of the probe when the probe with a certain length is rotating, and a single-mode optical fiber (1) that transmits optical signals is inserted in the spring tube (2);
a lens assembly, making the light from the optical fiber (1) to be focused at a position with a preset working distance, wherein the lens assembly comprises:

   a glass rod (3);
   a grin lens (4); and
   a reflector (5), wherein the reflector (5) changes forward light rays to lateral light rays, and the reflector (5) is a cylindrical-surface reflector;

**characterized in that**
the glass rod (3) is glued to the single-mode optical fiber (1), a gluing distance between the glass rod (3) and the single-mode optical fiber (1) is determined to achieve a working distance of the OCT microprobe;
the grin lens (4) is glued with the glass rod; and
wherein the glass rod (3) is configured to diffusely transmit the light, such that a clear aperture of the grin lens (4) is increased by the glass rod (3) glued between the single-mode optical fiber (1) and the grin lens (4), thereby increasing a numerical aperture of the OCT microprobe and to improve a transverse resolution of the OCT microprobe.

2. The OCT microprobe as claimed in claim 1, wherein an antireflective film is coated on a surface, in contact with air, of the grin lens (4), and is capable of reducing reflection of light rays between optical surfaces and enhancing light transmission performance.

3. The OCT microprobe as claimed in claim 2, wherein the surface, in contact with air, of the grin lens (4) is processed to be an inclined surface with an inclination angle 4° to 8°.

4. The OCT microprobe as claimed in claim 1, wherein a surface on which the glass rod (3) is glued with the single-mode optical fiber (1) is an inclined surface, to reduce interference of reflective light on signal light.

5. The OCT microprobe as claimed in claim 4, wherein an angle of the surface on which the glass rod (3) is glued with the single-mode optical fiber (1) is 4° to 12°.

6. The OCT microprobe as claimed in claim 1, further comprising:
a cylindrical inner tube passing through the OCT microprobe, wherein the reflector (5) is a cylindrical-surface reflector that is designed according to an inner diameter and an outer diameter of the cylindrical inner tube and a refractive index of material of the inner tube.

7. An OCT endoscopic scanning and imaging system comprising:

a sweep laser module, an interference module, a probe module, a data collection module, a data processing module, an image display module, an actuator, a balloon catheter, an inflation/deflation device and an OCT microprobe as claimed in any one of claims 1-6.

8. The OCT endoscopic scanning and imaging system as claimed in claim 7, wherein the actuator drives the OCT microprobe to rotately scan to generate 3D images.

9. A non-surgical imaging method for an OCT endoscopic imaging system, **characterized in that** the imaging method comprises steps:

a. placing an OCT microprobe as claimed in any one of claims 1-6 into an inner tube of a balloon catheter (10);
b. providing a light source to the single-mode optical fiber (1) through the lens assembly and focusing a facula at a predetermined working distance
c. collecting a returned optical signal; and
d. performing processing according to the returned optical signal to obtain OCT data.

**Patentansprüche**

1. Eine OCT-Mikrosonde, umfassend:

ein Federrohr (2), das in der Lage ist, ein ausreichendes Drehmoment bereitzustellen, um Synchronisation sowohl einer distalen Spitze als auch einer proximalen Spitze der Sonde aufrechtzuerhalten, wenn sich die Sonde mit einer bestimmten Länge dreht, und eine Einmoden-Glasfaser (1), die optische Signale überträgt, wird in das Federrohr (2) eingefügt;
eine Linsenanordnung, die das Licht von der Glasfaser (1) auf eine Position mit einem voreingestellten Arbeitsabstand fokussiert, wobei die Linsenanordnung umfasst:

einen Glasstab (3);
eine Grin-Linse (4); und
einen Reflektor (5), wobei der Reflektor (5) Vorwärtslichtstrahlen in seitliche Lichtstrahlen umwandelt, und der Reflektor (5) ein Reflektor mit zylindrischer Oberfläche ist;

**dadurch gekennzeichnet, dass**
der Glasstab (3) an die Einmoden-Glasfaser (1) geklebt ist, ein Klebeabstand zwischen dem Glasstab (3) und der Einmoden-Glasfaser (1) bestimmt ist, um einen Arbeitsabstand der OCT-Mikrosonde zu erreichen;
die Grin-Linse (4) mit dem Glasstab verklebt ist; und
wobei der Glasstab (3) so konfiguriert ist, dass er das Licht diffus durchlässt, so dass eine freie Apertur der Grin-Linse (4) durch den Glasstab (3), der zwischen die Einmoden-Glasfaser (1) und die Grin-Linse (4) geklebt ist, vergrößert wird, wodurch eine numerische Apertur der OCT-Mikrosonde erhöht und eine transversale Auflösung der OCT-Mikrosonde verbessert wird.

2. Die OCT-Mikrosonde nach Anspruch 1, wobei ein Antireflexionsfilm auf eine mit Luft in Kontakt stehende Oberfläche der Grin-Linse (4) aufgetragen ist, und in der Lage ist, die Reflektion von Lichtstrahlen zwischen optischen Oberflächen zu reduzieren und die Lichtdurchlässigkeitsleistung zu verbessern.

3. Die OCT-Mikrosonde nach Anspruch 2, wobei die mit Luft in Kontakt stehende Oberfläche der Grin-Linse (4) verarbeitet ist um eine geneigte Oberfläche mit einem Neigungswinkel von 4° bis 8° zu sein.

4. Die OCT-Mikrosonde nach Anspruch 1, wobei eine Oberfläche, auf die der Glasstab (3) mit der Einmoden-Glasfaser (1) geklebt ist, eine geneigte Oberfläche ist, um Interferenz von reflektierendem Licht auf Signallicht zu verringern.

5. Die OCT-Mikrosonde nach Anspruch 4, wobei ein Winkel der Oberfläche, auf die der Glasstab (3) mit der Einmoden-Glasfaser (1) geklebt ist, 4° bis 12° beträgt.

6. Die OCT-Mikrosonde nach Anspruch 1, ferner umfassend:
ein zylindrisches Innenrohr, das durch die OCT-Mikrosonde verläuft, wobei der Reflektor (5) ein Reflektor mit zylindrischer Oberfläche ist, der entsprechend einem Innendurchmesser und einem Außendurchmesser des zylindrischen Innenrohrs und einem Brechungsindex eines Materials des Innenrohrs ausgelegt ist.

7. Ein endoskopisches OCT- Scanning - und Bildgebungssystem, umfassend:
ein Rotationslasermodul, ein Interferenzmodul, ein Sondenmodul, ein Datenerfassungsmodul, ein Datenverarbeitungsmodul, ein Bildanzeigemodul, einen Aktuator, einen Ballonkatheter, eine Aufblas- / Abblasvorrichtung und eine OCT-Mikrosonde, nach einem der Ansprüche 1-6.

**8.** Das endoskopische OCT-Scanning - und Bildgebungssystem nach Anspruch 7, wobei der Aktuator die OCT-Mikrosonde antreibt, um rotierbar zu scannen, um 3D-Bilder zu erzeugen.

**9.** Ein nicht-chirurgisches Bildgebungsverfahren für ein endoskopisches OCT-Bildgebungssystem, das **dadurch gekennzeichnet ist, dass** das Bildgebungsverfahren Schritte umfasst:

> a. Platzieren einer OCT-Mikrosonde nach einem der Ansprüche 1 bis 6 in ein Innenrohr eines Ballonkatheters (10);
> b. Bereitstellen einer Lichtquelle für die Einmoden-Glasfaser (1) durch die Linsenanordnung und Fokussieren einer Facula in einem vorbestimmten Arbeitsabstand
> c. Sammeln eines zurückgegebenen optischen Signals; und
> d. Durchführen einer Verarbeitung entsprechend dem zurückgegebenen optischen Signal, um OCT-Daten zu erhalten.

**Revendications**

**1.** Une microsonde OCT comprenant :

> un tube ressort (2), capable de fournir suffisamment de couple pour maintenir la synchronisation d'une extrémité distale et d'une extrémité proximale lorsque la sonde d'une certaine longueur tourne, et une fibre optique monomode (1) qui transmet des signaux optiques étant insérée dans le tube ressort (2) ;
> un ensemble de lentille, concentrant la lumière de la fibre optique (1) sur une position avec une distance de travail prédéfinie, dans laquelle l'ensemble de lentille comprend :
>
>> une tige de verre (3) ;
>> une lentille à gradient d'indice (4) ;
>> un réflecteur (5), dans laquelle le réflecteur (5) change les rayons de lumière avant en rayons de lumière latéraux, et le réflecteur (5) est un réflecteur de surface cylindrique ;
>> **caractérisé en ce que**
>> la tige de verre (3) est collée sur la fibre optique monomode (1), une distance de collage entre la tige de verre (3) et la fibre optique monomode (1) étant déterminée pour obtenir une distance de travail de la microsonde OCT ;
>> la lentille à gradient d'indice (4) est collée à la tige de verre ; et
>> dans laquelle la tige de verre (3) est configurée pour transmettre la lumière de manière diffuse, de sorte que l'ouverture claire de la lentille à gradient d'indice (4) est augmentée par la tige de verre (3) collée entre la fibre optique monomode (1) et la lentille à gradient d'indice (4), augmentant ainsi l'ouverture numérique de la microsonde OCT et pour améliorer la résolution transversale de la microsonde OCT.

**2.** La microsonde OCT selon la revendication 1, dans laquelle un film anti-réfléchissant est posé sur une surface, en contact avec l'air, de la lentille à gradient d'indice (4) et est capable de réduire la réflexion des rayons lumineux entre les surfaces optiques et d'améliorer la performance de transmission de lumière.

**3.** La microsonde OCT selon la revendication 2, dans laquelle la surface, en contact avec l'air, de la lentille à gradient d'indice (4) est traitée pour être une surface inclinée avec un angle d'inclinaison de 4 à 8°.

**4.** La microsonde OCT selon la revendication 1, dans laquelle une surface sur laquelle la tige de verre (3) est collée à la fibre optique monomode (1) est inclinée pour réduire l'interférence de la lumière réfléchie sur le signal lumineux.

**5.** La microsonde OCT selon la revendication 4, dans laquelle un angle de la surface sur laquelle la tige de verre (3) est collée à la fibre de verre monomode (1) est de 4 à 12°.

**6.** La microsonde OCT selon la revendication 1, comprenant également :
un tube interne cylindrique passant à travers la microsonde OCT, dans laquelle le réflecteur (5) est un réflecteur à surface cylindrique conçu selon un diamètre interne et un diamètre externe du tube interne cylindrique et un indice de réfraction du matériau du tube interne.

**7.** Un système d'imagerie et de scanner endoscopique OCT comprenant :
un module de laser à balayage, un module d'interférence, un module de sonde, un module de collecte de données, un module de traitement des données, un module d'affichage d'image, un actionneur, un cathéter à ballonnet, un dispositif de gonflage/dégonflage et une microsonde OCT selon l'une quelconque des revendications 1 à 6.

**8.** Le système d'imagerie et de scanner endoscopique OCT selon la revendication 7, où l'actionneur pilote la microsonde OCT pour un scanner rotatif afin de générer des images 3D.

**9.** Une méthode d'imagerie non chirurgicale pour un système d'imagerie endoscopique OCT, **caractéri-**

sé en ce que la méthode d'imagerie comprend les étapes suivantes :

a. Placer une sonde OCT selon l'une quelconque des revendications 1 à 6 dans le tube interne d'un cathéter à ballonnet (10) ;

b. Fournir une source lumineuse pour la fibre optique monomode (1) à travers l'ensemble de lentille et concentrer une facule à une distance de travail prédéfinie ;

c. Collecter un signal optique de retour ;

d. Réaliser le traitement d'après le signal optique de retour pour obtenir les données OCT.

FIG.1

EP 3 295 988 B1

FIG.2A

FIG.2B

| SWEEP LASER MODULE | → | INTERFERENCE MODULE | → | PROBE MODULE | → | DATA COLLECTION MODULE | → | DATA PROCESSING MODULE | → | IMAGE DISPLAY MODULE |

| EXECUTION MECHANISM | → | OCT MICROPROBE AND BALLOON CATHETER | ← | NFLATION/ DEFLATION DEVICE |

FIG.3

FIG.4

FIG.5

FIG.6

FIG.7

FIG.8

FIG.9

```
┌──────────────┐    ┌──────────────┐    ┌──────────────┐
│ POWER        │    │ AIR PUMP AND │    │ CONTROL      │
│ SOURCE       │───▶│ ITS CONTROL  │◀──▶│ AND DISPLAY  │
│ PART         │    │ SYSTEM       │    │ PART         │
└──────────────┘    └──────────────┘    └──────────────┘
                            │
        ┌─────────┬─────────┼─────────┬─────────┬─────────┐
┌───────┴──┐ ┌────┴─────┐ ┌─┴──────┐ ┌┴────────┐ ┌───────┴──┐ ┌──┴───────┐
│ AIR      │ │ INFLATION│ │PRESSURE│ │ANTI-    │ │MECHANICAL│ │          │
│ PUMP     │ │/DEFLATION│ │SENSORS │ │EXPLOSION│ │PRESSURE  │ │THROTTLE  │
│          │ │SOLENOID  │ │        │ │PRESSURE │ │SWITCH    │ │VALVE     │
│          │ │VALVE     │ │        │ │SENSOR   │ │          │ │          │
└──────────┘ └──────────┘ └────────┘ └─────────┘ └──────────┘ └──────────┘
```

# FIG.10

FIG.11

FIG.12

FIG.13

| SWEEP LASER MODULE | → | OPTICAL CLOCK MODULE | → | DATA COLLECTION MODULE | → | DATA PROCESSING MODULE | → | IMAGE DISPLAY MODULE |

| EXECUTION MECHANISM | → | OCT MICROPROBE AND BALLOON CATHETER | ← | INFLATION/ DEFLATION DEVICE |

FIG.14

FIG.15

EP 3 295 988 B1

| | SYNCHRONIZATION | | SYNCHRONIZATION | | SYNCHRONIZATION |
|---|---|---|---|---|---|
| TRANSMISSION | ORIGINAL SIGNAL TRANSMISSION (THE n-1$^{\text{TH}}$ FRAME) | | ORIGINAL SIGNAL TRANSMISSION (THE n$^{\text{TH}}$ FRAME) | | ORIGINAL SIGNAL TRANSMISSION (THE n+1$^{\text{TH}}$ FRAME) | |
| PROCESSING | DIGITAL SIGNAL PROCESSING (THE n-2$^{\text{TH}}$ FRAME) | | DIGITAL SIGNAL PROCESSING (THE n-1$^{\text{TH}}$ FRAME) | WAIT | DIGITAL SIGNAL PROCESSING (THE n$^{\text{TH}}$ FRAME) |

FIG.16

9

10

13

14

FIG.17

FIG.18

EP 3 295 988 B1

FIG.19

FIG.20

FIG.21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008137710 A1
- WO 2009140617 A2
- JP 200720649 A
- US 2013204126 A1
- US 2010030144 A1
- US 2007219451 A1
- US 2004062890 A1
- US 2010292536 A1
- US 2013044330 A1
- US 2007265521 A1

**Non-patent literature cited in the description**

- Influence of optical probe packaging on a 3D MEMS scanning micro-mirror for optical coherence tomography (OCT) applications. **PREMACHANDRAN C S.** 58th Electronic Components and Technology Conference 27-30 May 2008. IEEE, 27 May 2008, 823-833
- **JIEFENG XI et al.** Generic real-time uniform K-space sampling method for high-speed swept-Source optical coherence tomography. *OPTICS EXPRESS,* 26 April 2010, vol. 18 (9), 9511